# FASCICULE DE BREVET EUROPEEN

(11) **EP 2 292 778 B1**
(45) Date de publication et mention de la délivrance du brevet: **14.09.2016**
(21) Numéro de dépôt: 10172183.5
(22) Date de dépôt: 23.04.1999
(51) Int. Cl.: C12N 7/00, C12N 15/86, A61K 48/00

(54) **Utilisation de séquences lentivirales de structure triplex pour l'import nucléaire de séquences nucléotidiques**
Verwendung von Lentiviralensequenzen mit Triplex-Structur zum Kerntransfer von Nukleotidsequenzen
Use of lentiviral triplex forming sequences for nuclear transfer of nucleotide sequences

(30) Priorité: 24.04.1998 FR 9805197
(43) Date de publication de la demande: 09.03.2011
(62) Demande divisionnaire de: 99915829.8
(73) Titulaire: Institut Pasteur, 75724 Paris Cédex 15 (FR); Centre National de la Recherche Scientifique, 75794 Paris Cedex 16 (FR); Institut National de la Santé et de la Recherche Médicale, 75654 Paris Cedex 13 (FR)
(72) Inventeur: Charneau, Pierre, 75005 Paris (FR); Zennou, Véronique, 75002 Paris (FR); Firat, Hüseyin, 68330 Huningue (FR)
(74) Mandataire: Desaix, Anne

(56) Documents cités:
- EP-A- 0 611 822
- WO-A-97/12622
- WO-A-97/32983
- WO-A-98/39463
- REISER J ET AL: "TRANSDUCTION OF NONDIVIDING CELLS USING PSEUDOTYPED DEFECTIVE HIGH-TITER HIV TYPE 1 PARTICLES", PROCEEDINGS OF THE NATIONAL ACADEMY OF SCIENCES OF THE UNITED STATES (PNAS), NATIONAL ACADEMY OF SCIENCE, US, vol. 93, 1 décembre 1996 (1996-12-01), pages 15266-15271, XP002915814, ISSN: 0027-8424, DOI: DOI:10.1073/PNAS.93.26.15266
- SIRVEN A, PFLUMIO F, ZENNOU V, TITEUX M, VAINCHENKER W, COULOMBEL L, DUBART-KUPPERSCHMITT A, CHARNEAU P.: "The human immunodeficiency virus type-1 central DNA flap is a crucial determinant for lentiviral vector nuclear import and gene transduction of human hematopoietic stem cells", BLOOD., vol. 96, no. 13, 15 décembre 2000 (2000-12-15), pages 4103-4010, XP002168293,
- CHARNEAU, P. ET AL.: "HIV-1 Reverse transcription", JOURNAL OF MOLECULAR BIOLOGY, vol. 241, no. 5, 2 septembre 1994 (1994-09-02), pages 651-662, XP002090794,
- CHARNEAU, P. & CLAVEL, F.: "A single-stranded gap in Human Immunodeficiency Virus unintegrated linear DNA defined by a central copy of polypurine tract", JOURNAL OF VIROLOGY., vol. 65, no. 5, mai 1991 (1991-05), pages 2415-2421, XP002090795, ICAN SOCIETY FOR MICROBIOLOGY US
- ZENNOU V ET AL: "HIV-1 genome nuclear import is mediated by a central DNA flap", CELL, CELL PRESS, CAMBRIDGE, NA, US, vol. 101, no. 2, 14 avril 2000 (2000-04-14), pages 173-185, XP002168292, ISSN: 0092-8674, DOI: DOI:10.1016/S0092-8674(00)80828-4
- CHARNEAU, P. ET AL.: "A second origin of DNA plus-strand synthesis is required for optimal Human Immunodeficiency Virus replication", JOURNAL OF VIROLOGY., vol. 66, no. 5, mai 1992 (1992-05), pages 2814-2820, XP002090796, ICAN SOCIETY FOR MICROBIOLOGY US
- HUANG S ET AL: "LYMPHOID AND MYELOID DIFFERENTIATION OF SINGLE HUMAN CD34+, HLA-DR+, CD38- HEMATOPOIETIC STEM CELLS", BLOOD, AMERICAN SOCIETY OF HEMATOLOGY, US, vol. 83, no. 6, 15 mars 1994 (1994-03-15), pages 1515-1526, XP000608633, ISSN: 0006-4971
- UCHIDA N ET AL: "HIV, but not murine leukemia virus, vectors mediate high efficiency gene transfer into freshly isolated G0/G1 human hematopoietic stem cells", PROCEEDINGS OF THE NATIONAL ACADEMY OF SCIENCES OF THE UNITED STATES (PNAS), NATIONAL ACADEMY OF SCIENCE, US, vol. 95, no. 20, 29 septembre 1998 (1998-09-29), pages 11939-11944, XP002215385, ISSN: 0027-8424, DOI: DOI:10.1073/PNAS.95.20.11939
- ZUFFEREY R ET AL: "MULTIPLY ATTENUATED LENTIVIRAL VECTOR ACHIEVES EFFICIENT GENE DELIVERY IN VIVO", NATURE BIOTECHNOLOGY, vol. 15, 1 septembre 1997 (1997-09-01), pages 871-875, XP002056816, ISSN: 1087-0156
- KIM SY, BYRN R, GROOPMAN J, BALTIMORE D.: "Temporal aspects of DNA and RNA synthesis during human immunodeficiency virus infection: evidence for differential gene expression", J VIROL, vol. 63, no. 9, septembre 1989 (1989-09), pages 3708-3713, XP002615707,
- ERLWEIN, O. ET AL.: "Sequences in pol are required for transfer of Human Foamy Virus-based vectors", JOURNAL OF VIROLOGY., vol. 72, no. 7, juillet 1998 (1998-07), pages 5510-5516, XP002090797, ICAN SOCIETY FOR MICROBIOLOGY US
- MICHAEL P. SHERMAN ET AL: 'Slipping through the door: HIV entry into the nucleus' MICROBES AND INFECTION vol. 4, no. 1, 01 Janvier 2002, pages 67 - 73, XP055179495 DOI: 10.1016/S1286-4579(01)01511-8 ISSN: 1286-4579

### SEQUENCE LISTING

<110> INSTITUT PASTEUR
<120> UTILISATION DE SEQUENCES D'ADN DE STRUCTURE TRIPLEX POUR LE TRANSFERT DE SEQUENCES NUCLEOTIDIQUES
<130> B03920AAB AD/LV/CAL
<150> FR9805197
   <151> 1998-04-24
<160> 39
<170> PatentIn version 3.3
<210> 1
   <211> 14
   <212> DNA
   <213> Artificiel
<220>
   <223> oligonucléotide
<400> 1
   aacaaaggga ggga 14
<210> 2
   <211> 24
   <212> DNA
   <213> Artificial
<220>
   <223> oligonucléotide
<400> 2
   aaaaaatttt gtttttacaa aatc 24
<210> 3
   <211> 23
   <212> DNA
   <213> Artificial
<220>
   <223> oligonucléotide
<400> 3
   ccggatcccc accggtcgcc acc 23
<210> 4
   <211> 23
   <212> DNA
   <213> Artificial
<220>
   <223> oligonucléotide
<400> 4
   ccctcgagct agagtcgcgg ccg 23
<210> 5
   <211> 38
   <212> DNA
   <213> Artificial
<220>
   <223> oligonucléotide
<400> 5
   gtcgtcggcg ccgaattcac aaatggcagt attcatcc 38
<210> 6
   <211> 34
   <212> DNA
   <213> Artificial
<220>
   <223> oligonucléotide
<400> 6
   gtcgtcggcg ccccaaagtg gatctctgct gtcc 34
<210> 7
   <211> 32
   <212> DNA
   <213> Artificial
<220>
   <223> oligonucléotide
<400> 7
   ccagatctac gcgtgccacc atggctgctg gt 32
<210> 8
   <211> 27
   <212> DNA
   <213> Artificial
<220>
   <223> oligonucléotide
<400> 8
   cggaattcga cctaaacgca acggatg 27
<210> 9
   <211> 200
   <212> DNA
   <213> Caprine arthritis-encephalitis virus
<220>
   <221> misc_feature
   <222> (1)..(200)
   <223> Polynucléotide comprenant les régions cPPT et CTS
<400> 9
   gttccagcca caatttgtcg ctgtagaatc agccatagca gcagccctag tcgccataaa 60
   tataaaaaga aagggtgggc tggggacaag ccctatggat atttttatat ataataaaga 120
   acagaaaaga ataaataata aatataataa aaattctcaa aaaattcaat tctgttatta 180
   cagaataagg aaaagaggac 200
<210> 10
   <211> 200
   <212> DNA
   <213> Equine infectious anemia virus
<220>
   <221> misc_feature
   <222> (1)..(200)
   <223> Polynucléotide comprenant les régions cPPT et CTS
<400> 10
   cttgtaacaa agggagggaa agtatgggag gacagacacc atgggaagta tttatcacta 60
   atcaagcaca agtaatacat gagaaacttt tactacagca agcacaatcc tccaaaaaat 120
   tttgttttta caaaatccct ggtgaacatg attggaaggg acctactagg gtgctgtgga 180
   agggtgatgg tgcagtagta 200
<210> 11
   <211> 200
   <212> DNA
   <213> Visna virus
<220>
   <221> misc_feature
   <222> (1)..(200)
   <223> Polynucléotide comprenant les régions cPPT et CTS
<400> 11
   ggaccctcat tactctaaat ataaaaagaa agggtgggct agggacaagc cctatggata 60
   tatttatatt taataaggaa caacaaagaa tacagcaaca aagtaaatca aaacaagaaa 120
   aaattcgatt ttgttattac agaacaagaa aaagagggca tccaggagag tggcaaggac 180
   caacacaggt actttggggc 200
<210> 12
   <211> 200
   <212> DNA
   <213> Simian immunodeficiency virus
<220>
   <221> misc_Feature
   <222> (1)..(200)
   <223> Polynucléotide comprenant les régions cPPT et CTS
<400> 12
   tactgatggc ttgcatactt cacaatttta aaagaaaggg aggaataggg ggacagactt 60
   cagcagagag actaattaat ataataacaa cacaattaga aatacaacat ttacaaacca 120
   aaattcaaaa aattttaaat tttagagtct actacagaga agggagagac cctgtgtgga 180
   aaggaccggc acaattaatc 200
<210> 13
   <211> 200
   <212> DNA
   <213> Human immunodeficiency virus
<220>
   <221> misc_feature
   <222> (1)..(200)
   <223> Polynucléotide comprenant les régions cPPT et CTS
<400> 13
   tgcatgaatt ttaaaagaag ggggggaata ggggatatga ctccatcaga aagattaatc 60
   aatatgatca ccacagaaca agagatacaa ttcctccaag ccaaaaattc aaaattaaaa 120
   gattttcggg tctatttcag agaaggcaga gatcagttgt ggaaaggacc tggggaacta 180
   ctgtggaaag gagaaggagc 200
<210> 14
   <211> 200
   <212> DNA
   <213> Human immunodeficiency virus
<220>
   <221> misc_feature
   <222> (1)..(200)
   <223> Polynucléotide comprenant les régions cPPT et CTS
<400> 14
   cagtattcat ccacaatttt aaaagaaaag gggggattgg ggggtacagt gcaggggaaa 60
   gaatagtaga cataatagca acagacatac aaactaaaga attacaaaaa caaattacaa 120
   aaattcaaaa ttttcgggtt tattacaggg acagcagaga tccactttgg aaaggaccag 180
   caaagctcct ctggaaaggt 200
<210> 15
   <211> 119
   <212> DNA
   <213> Human immunodeficiency virus
<220>
   <221> misc_feature
   <222> (1)..(119)
   <223> Polynucléotide comprenant les régions cPPT et CTS
<400> 15
   ttttaaaaga aaagggggga ttggggggta cagtgcaggg gaaagaatag tagacataat 60
   agcaacagac atacaaacta aagaattaca aaaacaaatt acaaaaattc aaaattttc 119
<210> 16
   <211> 15
   <212> DNA
   <213> Human immunodeficiency virus
<400> 16
   aaaagaaaag ggggg 15
<210> 17
   <211> 14
   <212> DNA
   <213> Human immunodeficiency virus
<400> 17
   aaaacaaggg gggg 14
<210> 18
   <211> 15
   <212> DNA
   <213> Simian immunodeficiency virus
<400> 18
   aaaagaaaag ggggg 15
<210> 19
   <211> 15
   <212> DNA
   <213> Simian immunodeficiency virus
<400> 19
   aaaagaaaag ggagg 15
<210> 20
   <211> 22
   <212> DNA
   <213> visna virus
<400> 20
   aaaaagaaaa aagaaagggt gg 22
<210> 21
   <211> 22
   <212> DNA
   <213> caprine arthritis-encephalitis virus
<400> 21
   aaaaataaaa aaagaaaggg tg 22
<210> 22
   <211> 13
   <212> DNA
   <213> Equine infectious anemia virus
<400> 22
   aacaaggggg gaa 13
<210> 23
   <211> 14
   <212> DNA
   <213> Human immunodeficiency virus
<400> 23
   aaaagaaggg gggg 14
<210> 24
   <211> 15
   <212> DNA
   <213> simian immunodeficiency virus
<400> 24
   aaaagaaggg gagga 15
<210> 25
   <211> 15
   <212> DNA
   <213> Simian immunodeficiency virus
<400> 25
   aaaagaaagg gagga 15 Page 5
<210> 26
   <211> 22
   <212> DNA
   <213> visna virus
<400> 26
   aaatataaaa aagaaagggt gg 22
<210> 27
   <211> 22
   <212> DNA
   <213> Caprine arthritis-encephalitis virus
<400> 27
   aaatataaaa aaagaaaggg tg 22
<210> 28
   <211> 13
   <212> DNA
   <213> Equine infectious anémia virus
<400> 28
   aaagggaggg aaa 13
<210> 29
   <211> 9
   <212> PRT
   <213> Artificial
<220>
   <223> peptide
<400> 29
   Lys Thr Trp Gly Gln Tyr Trp Gln Val
   1 5
<210> 30
   <211> 9
   <212> PRT
   <213> Artificial
<220>
   <223> peptide
<400> 30
   Ile Thr Asp Gln Val Pro Phe Ser Val
   1 5
<210> 31
   <211> 9
   <212> PRT
   <213> Artificial
<220>
   <223> peptide
<400> 31
   Tyr Leu Glu Pro Gly Pro Val Thr Ala
   1 5
<210> 32
   <211> 10
   <212> PRT
   <213> Artificial
<220>
   <223> peptide
<400> 32
   Leu Leu Asp Gly Thr Ala Thr Leu Arg Leu
   1               5                   10
<210> 33
   <211> 9
   <212> PRT
   <213> Artificial
<220>
   <223> peptide
<400> 33
   Ala Ala Gly Ile Gly Ile Leu Thr Val
   1               5
<210> 34
   <211> 9
   <212> PRT
   <213> Artificial
<220>
   <223> peptide
<400> 34
   Ile Leu Thr Val Ile Leu Gly Val Leu
   1               5
<210> 35
   <211> 9
   <212> PRT
   <213> Artificial
<220>
   <223> peptide
<400> 35
   Met Leu Leu Ala Val Leu Tyr Cys Leu
   1               5
<210> 36
   <211> 9
   <212> PRT
   <213> Artificial
<220>
   <223> peptide
<400> 36
   Tyr Met Asp Gly Thr Met Ser Gln Val
   1 5
<210> 37
   <211> 9
   <212> PRT
   <213> Artificial
<220>
   <223> peptide
<400> 37
   Val Leu Pro Asp Val Phe Ile Arg Cys
   1 5
<210> 38
   <211> 9
   <212> PRT
   <213> Artificial
<220>
   <223> peptide
<400> 38
   Phe Leu Trp Gly Pro Arg Ala Leu Val
   1 5
<210> 39
   <211> 92
   <212> PRT
   <213> Artificial
<220>
   <223> peptide
<400> 39
   Ala Ala Gly Ile Gly Ile Leu Thr Val Phe Leu Trp Gly Pro Arg Ala
   1               5                   10                  15
   Leu Val Met Leu Leu Ala Val Leu Tyr Cys Leu Leu Leu Asp Gly Thr
               20                  25                  30
   Ala Thr Leu Arg Leu Lys Thr Trp Gly Gln Tyr Trp Gln Val Tyr Met
           35                  40                  45
   Asp Gly Thr Met Ser Gln Val Ile Thr Asp Gln Val Pro Phe Ser Val
       50                  55                  60
   Tyr Leu Glu Pro Gly Pro Val Thr Ala Ile Leu Thr Val Ile Leu Gly
   65                  70                  75                  80
   Val Leu Val Leu Pro Asp Val Phe Ile Arg Cys Val
                   85                  90

## Description

La présente demande a pour objet l'utilisation de séquences d'ADN susceptibles de présenter une structure ou organisation à trois brins (appelée "ADN triplex"), pour le transfert de séquences de nucléotides dans des cellules, ainsi que des vecteurs recombinants contenant ces séquences triplex.

L'invention concerne l'utilisation d'un polynucléotide pour la fabrication d'un plasmide vecteur recombinant destiné à la production, par cotransfection de plasmides de transcomplémentation, de particules lentivirales recombinantes dépourvues de gènes lentiviraux et destinées à la transductions dans des cellules cibles eucaryotes d'une séquence de nucléotides d'intérêt, particules dans lesquelles ledit polynucléotide est le déterminant d'import nucléaire, ledit polynucléotide étant dérivé d'un lentivirus et constitué d'une région cis-active d'initiation centrale (cPPT) contenant au moins 10 nucléotides, d'une région cis-active de terminaison (CTS) contenant au moins 10 nucléotides, et d'un enchaînement interne de nucléotides encadré par ces deux régions, ladite région CTS et ledit enchaînement interne de nucléotide constituant une séquence ayant une taille de 80 à 120 nucléotides, ledit polynucléotide étant capable de former une structure d'ADN triplex lors de la rétrotranscription du génome desdites particules dans la cellule cible eucaryote, ledit plasmide étant dépourvu de gènes lentiviraux et comprenant une séquence de nucléotides recombinante comprenant (i) ladite séquence de nucléotides d'intérêt placée sous le contrôle de signaux régulateurs de transcription et d'expression, (ii) des signaux régulateurs de rétrotranscription, d'expression et d'encapsidation dérivés d'un lentivirus et (iii) ledit polynucléotide, les susdites régions cis-actives étant insérées dans une orientation fonctionnelle avec les susdits signaux régulateurs dérivés d'un lentivirus.

La demande a donc pour objet la définition et la fourniture de nouveaux moyens susceptibles d'être mis en oeuvre par exemple dans le cadre de protocoles de thérapie génique ou de transgénèse pour la création d'animaux ou de plantes transgéniques ou encore de cellules ou lignées cellulaires recombinantes. Ces moyens comprennent l'élaboration de nouveaux vecteurs susceptibles de transférer une séquence de nucléotides, et en particulier un séquence d'intérêt thérapeutique, dans des cellules cibles du corps humain ou animal.

Une limitation importante des approches de thérapie génique connues jusqu'à ce jour, réside dans la vectorisation du gène d'intérêt thérapeutique. Les vecteurs rétroviraux dérivés d'oncovirus, principalement du MoMLV, ont été largement utilisés pour le transfert de gène. Leur application est grandement limitée par le fait que les oncovirus ne s'intègrent que dans des cellules cibles en division active. Au contraire, les lentivirus ont la capacité unique, parmi les rétrovirus, d'infecter des cellules différenciées, non mitotiques, et représentent des candidats viraux intéressants pour le développement de nouveaux vecteurs. Tout en conservant les avantages d'un vecteur oncoviral (absence d'immunogénicité, intégration stable), les lentivirus pourraient permettre la transduction *in vivo* de tissus différenciés non mitotiques (cerveau, muscle, foie, poumon...) et trouver ainsi un champ d'applications considérable en thérapie génique.

Différentes tentatives de construction de vecteurs rétroviraux à partir de lentivirus ont été rapportées. On citera à cet égard les travaux de Poznansky M. et al (J. Virol 1991, 65, 532-6), de Naldini et al (Science, 1996, 272, p 263-7) réalisés à partir du rétrovirus HIV et ceux de Poeschla EM et al (Nature Medecine, 1998, 4, p 354-7) réalisés à partir du rétrovirus FIV.

Reiser J. et al (PNAS 1996, 93, 15266-71) décrit la construction de vecteurs lentiviraux dérivés de HIV-1 et délétés d'une partie du gène env qui est remplacée par une séquence de nucléotides d'intérêt liée codant pour les gènes marqueurs Neo ou HSA. Ces vecteurs conservant les séquences LTR gag pol de HIV-1 comprennent les séquences correspondant aux gènes pol et vif intacts. Ces vecteurs sont utilisés pour le transfert des séquences lentivirales et marqueur dans des cellules mitotiques ou non mitotiques. L'expression des marqueurs et l'analyse de l'ADN démontrent que les séquences correspondantes ont été intégrées dans le génome contenu dans le noyau, et donc importées dans le noyau préalablement.

Les inventeurs ont recherché des déterminants impliqués dans le mécanisme d'entrée du génome rétroviral dans le noyau des cellules (mécanisme d'import nucléaire) infectées.

L'identification d'un déterminant ADN triplex essentiel à l'import a conduit les inventeurs à définir de nouveaux moyens, et en particulier des vecteurs utilisables pour le transfert de gènes, ou plus généralement de séquences de nucléotides (désignés dans la suite par l'expression "transgènes") dans des cellules cibles. Les inventeurs ont en particulier travaillé à partir du rétrovirus HIV ("human immunodeficiency virus", ou VIH), membre de la famille des lentivirus, et ont identifié et isolé un déterminant viral responsable de l'import nucléaire de l'ADN proviral de HIV dans les cellules cibles : l'ADN triplex central. Cet ADN triplex s'est avéré pouvoir fonctionner dans des vecteurs, hors du contexte naturel du génome HIV1, comme déterminant d'import nucléaire permettant l'entrée du génome vecteur dans le noyau de cellules cibles.

Les mécanismes d'entrée de l'ADN rétroviral dans le noyau comportent des différences considérables d'une famille rétrovirale à une autre. Le génome des lentivirus est capable de traverser la membrane nucléaire du noyau interphasique via l'adressage puis la translocation de son complexe de pré-intégration (ADN linéaire et protéines associées) à travers le pore nucléaire. Ainsi, ces virus sont capables de se répliquer en absence de division de la cellule cible. Ils infectent en particulier les macrophages tissulaires différenciés et les cellules dendritiques, cellules au centre de la transmission, de la dissémination, et de la physiopathologie du HIV. A l'inverse, les génomes des oncovirus et des spurnavirus sont incapables de traverser la barrière représentée par la membrane nucléaire. Leur complexe de pré-intégration doit attendre la mitose, et la désorganisation de la membrane nucléaire, pour accéder aux chromosomes mitotiques et s'intégrer.

Les déterminants viraux responsables de l'import nucléaire de l'ADN du virus HIV1 ont été recherchés par les inventeurs. L'identification et la compréhension fonctionnelle des mécanismes moléculaires de l'import nucléaire du complexe de pré-intégration de HIV présente en effet des intérêts fondamentaux importants. Les inventeurs ont identifié un mécanisme original d'import nucléaire du génome HIV-1 selon lequel cet import serait gouverné par une structure ADN, un triplex au centre des molécules d'ADN linéaires, généré par des étapes particulières à la rétrotranscription lentivirale.

La structure d'ADN triplex présente au centre des molécules d'ADN linéaires générées lors de la rétrotransciption lentivirale, en particulier dans le rétrovirus HIV, a été décrite par les inventeurs dans différentes publications antérieures (Charneau P. et al., J. Mol. Biol. 1994, 241, 651-662 Carneau P. et al, Journal of Virology, Mai 1991, p. 2415-2421 ; Charneau P. et al, Journal of Virology, 1992, vol. 66, p. 2814-2820.

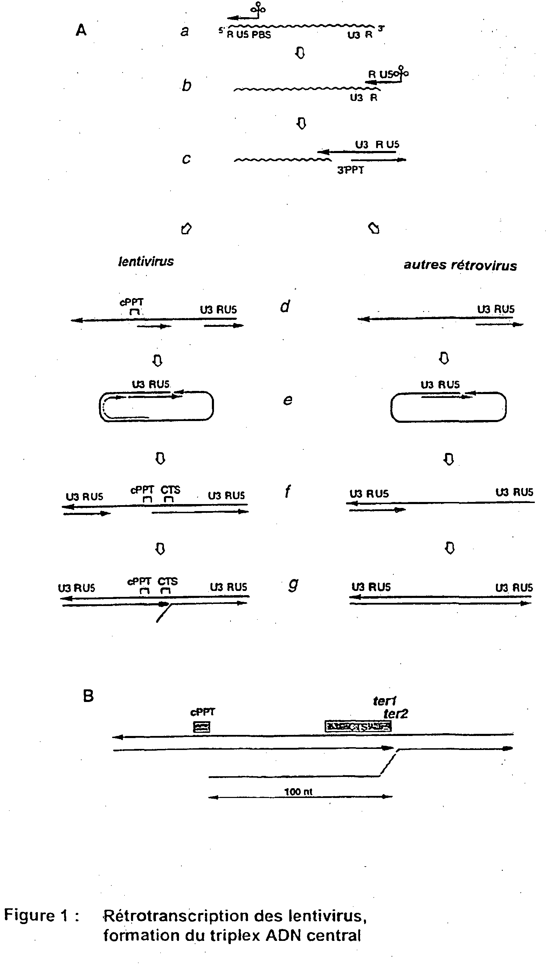

La structure d'ADN formant un triplex lors de la rétrotranscription virale, est un polynucléotide comportant une région cis- active d'initiation centrale, ou polypurine tract (cPPT), et une région cis- active de terminaison (CTS), ces régions permettant l'initiation de la transcription d'un brin+ dont la synthèse est initiée par la région PPT présente au centre du génome de HIV ou d'autres lentivirus, et l'interruption de la synthèse d'un second brin+, dont la synthèse est initiée au niveau d'un site PPT 3' en amont du LTR rétroviral (figure 1).

La formation de la structure d'ADN triplex est la conséquence d'un événement de déplacement de brin discret au sein du génome du rétrovirus, bloqué par la séquence CTS (Charneau et al, J. Mol. Biol., 1994).

Il est entendu que le terme "ADN triplex" utilisé ici désigne une région d'ADN à trois brins, sans référence à la structuration de ces brins entre eux (brin déplacé libre, ou formant une triple hélice ou une D-loop... etc).

La structure d'ADN triplex ainsi formée lors de la rétrotranscription permet, ou à tout le moins contribue à l'entrée du génome lentiviral dans le noyau de la cellule, permettant de ce fait l'infection de cellules non mitotiques.

A partir de l'identification de ce mécanisme requis pour l'entrée de lentivirus dans le noyau des cellules cibles, les inventeurs ont élaboré une nouvelle génération de vecteur lentiviral, incluant la région ADN triplex. L'introduction d'un fragment d'ADN, issu du génome HIV-1 et comprenant les séquences cPPT et CTS actives en cis, dans un système de vecteur HIV, augmente la transduction de gènes dans les cellules en stimulant le taux d'import nucléaire de l'ADN vecteur. Cette génération de vecteurs lentiviraux à triplex améliore notablement la transduction de gène dans des cellules, mitotiques ou non.

La demande décrit une séquence nucléotidique d'origine lentivirale ou lentiviraloïde pouvant être préparée de manière synthétique, comprenant des régions cPPT et CTS actives en cis dans la rétrotranscription en général, et en particulier 2 polynucléotides associés lorsqu'ils sont placés dans le génome lentiviral normal, ces polynucléotides contenant au moins 10 nucléotides chacun.

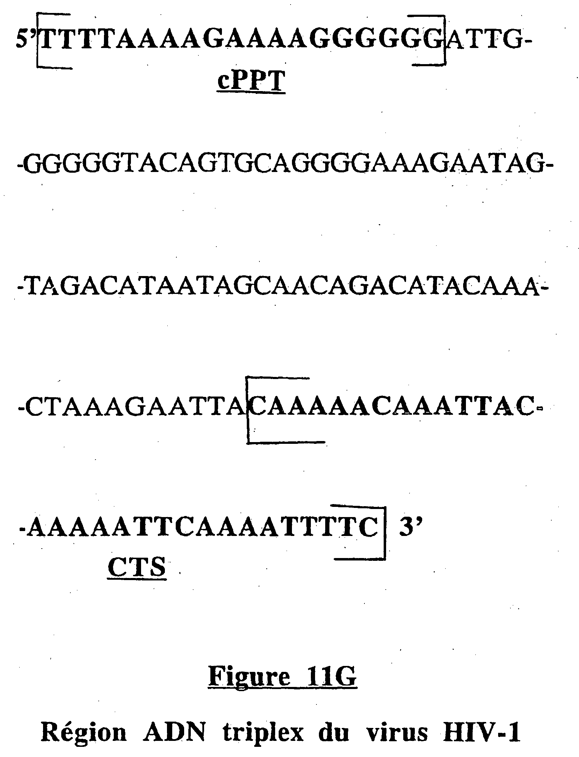

La séquence nucléotidique utilisée dans l'invention comprend (voir figure 11G où les séquences d'intérêt cis actives sont encadrées) d'un côté une séquence nucléotidique courte appelée "cPTT" dans le cas d'HIV1 (au minimum 10 paires de bases) et de l'autre une séquence appelée "CTS" d'au moins 10 paires de bases dans le cas d'HIV1. Les deux séquences actives en cis et une séquence nucléotidique issue du génome rétroviral et située entre ces deux séquences cis correspondent à environ 120 nucléotides dans le cas du génome d'HIV1 naturel.

La demande décrit également une séquence nucléotidique comprenant trois brins d'ADN constitués par d'une part, la région CTS (ou une région équivalente dans le cas d'une origine du génome utilisé autre que celle d'HIV1 mais ayant les mêmes propriétés que la région CTS publiée par Charneau et al, J. Mol. Biol., 1994, et d'autre part, en amont de CTS, une région ayant environ 90 à 110 nucléotides, de préférence 99 nucléotides dans le cas d'HIV1.

La demande décrit un polynucléotide comprenant un fragment d'ADN à deux brins correspondant à la région cPPT ("polypurine track") associée à une séquence polynucléotidique présente naturellement dans le génome d'HIV1 (ou une séquence équivalente naturelle ou synthétique) et enfin une région CTS nucléotidique qui adopte une conformation qui définit la fin de la région à trois brins (côté 3') après rétrotranscription.

Cette conformation à trois brins est dénommée "séquence triplex".

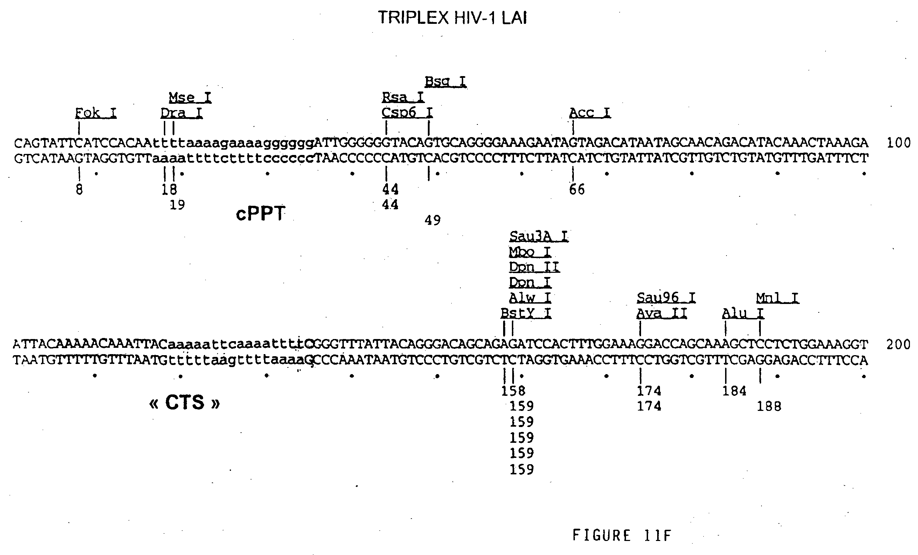

A titre d'exemple, la séquence triplex est celle figurant sur la figure 11 F pour HIV1 ou sur la figure 11G. Le triplex, *in vivo,* lorsqu'il est présent dans un vecteur utilisable pour la pénétration des membranes nucléaires des cellules eucaryotes, stimule l'importation d'ADN dans le noyau de la cellule à modifier ou à transduire.

La demande décrit l'utilisation de cette séquence triplex seule ou dans un vecteur pour introduire des séquences nucléotidiques auxquelles la séquence triplex est liée dans le noyau de la cellule eucaryote réceptrice.

La demande décrit ainsi un vecteur recombinant caractérisé en ce qu'il comprend un polynucléotide comportant une région cis-active d'initiation centrale (cPPT) et une région cis-active de terminaison (CTS), ces régions étant d'origine lentivirale ou lentiviraloïde, ledit vecteur comprenant en outre une séquence de nucléotides déterminée (transgène ou séquence d'intérêts et des signaux de régulation de rétrotranscription, d'expression et d'encapsidation d'origine lentivirale ou lentiviraloïde.

Le terme "polynucléotide" utilisé se rapporte à toute séquence d'acide nucléique, sous forme de simple brin ou de double brin ou triple brin, qu'il s'agisse d'ADN par exemple d'ADNc, ou d'ARN.

A titre d'exemple, la demande décrit le transfert de transgènes à visée thérapeutique, en particulier dans le cadre de protocoles de thérapie génique somatique, pour insérer une séquence de nucléotides modulatrice ou réparatrice d'une activité déficiente dans les cellules somatiques d'un organisme afin de remédier au mauvais fonctionnement d'un gène endogène, ou pour permettre l'expression d'une fonction supplémentaire, notamment d'une fonction suppressive de l'expression ou de l'activité d'un gène, dans un but thérapeutique.

Par l'expression "thérapeutique", on entend la recherche ou l'obtention d'un effet préventif ou curatif, ou encore la recherche ou l'obtention d'une amélioration ou d'une stabilisation de l'état pathologique d'un patient.

Dans le cadre de l'invention, et à titre d'exemple, les séquences nucléotidiques dites transgènes ou séquences nucléotidiques d'intérêt peuvent donc être des gènes ou partie de gènes ou séquences dérivées de gènes, par exemple des ADNc ou des ARN. II peut aussi s'agir de séquences antisens, de séquences mutants négatifs d'un gène donné, ou de séquences intervenant dans les fonctions de transcription, d'expression, d'activation de gènes, ou encore des séquences appropriées pour l'activation de prodrogues ou de substances cytotoxiques.

Les séquences de transgènes dans le cadre de l'invention peuvent aussi avoir une activité de stimulation ou d'induction de la réponse immunitaire, cellulaire ou humorale, par exemple lorsqu'elles sont utilisées pour transformer des cellules présentatrices de l'antigène.

Ainsi, l'invention peut être appliquée à la préparation de vecteurs utilisables en vue d'une thérapie génique dans des domaines variés tels que celui des maladies héréditaires comportant l'altération d'un gène, ces maladies comprenant par exemple la myopathie de Duchenne, la mucoviscidose, des maladies neuro-dégénératives ou encore des pathologies acquises telles que des pathologies malignes conduisant naturellement à une réponse faible du système immunitaire. L'invention permet aussi d'envisager des traitements d'immunothérapie pour stimuler la réponse à des agents pathogènes, par exemple par la production de CTL, par exemple dans le cas de pathologies telles que les cancers ou des pathologies telles que le SIDA, ou diminuer la réponse contre les antigènes du soi dans le cas des maladies auto-immunes.

La demande décrit également la fourniture de moyens permettant l'élaboration de compositions immunogènes ou de vaccins, prophylactiques ou thérapeutiques ou de compositions immunogènes.

Les vecteurs lentiviraux contenant un ADN triplex selon la divulgation sont également utilisés pour la construction d'animaux transgéniques, par la transduction de gènes dans des lignées ou des cellules embryonnaires.

Le vecteur utilisé dans l'invention contient un transgène inséré sous le contrôle de séquences régulatrices de transcription ou d'expression, virales ou non.

Le transgène peut être inclus dans une cassette d'expression comprenant les séquences appropriées pour la régulation de son expression dans une cellule.

Le vecteur recombinant est caractérisé en ce que les séquences d'origine rétrovirales qu'il contient sont dérivées du génome d'un lentivirus.

Dans le cadre de la présente demande, le terme "dérivé" englobe toute séquence identique à la séquence contenue dans le génome du rétrovirus, toute séquence modifiée par mutation, insertion, délétion, recombinaison, dès lors qu'elle conserve la fonction essentielle qu'elle possède au sein du génome rétroviral en vue de son insertion au sein du vecteur de l'invention.

Une telle séquence pourra être obtenue par tout moyen en soi connu permettant l'identification et l'isolement de séquences de nucléotides à partir de leur organisme d'origine, en particulier comprenant des étapes de clonage et/ou d'amplification, ou par synthèse selon toute technique disponible.

Le vecteur recombinant ainsi décrit peut par exemple être un plasmide recombiné par une construction lentivirale ou lentiviraloïde et un transgène, le cas échéant contenu dans une cassette d'expression.

Un tel vecteur est utilisable pour la transduction de cellules, et en particulier de cellules d'encapsidation ou/et de cellules cibles, par toute méthode en soi connue, incluant la transfection ou l'infection ou la transduction par exemple par un vecteur adénovirus ou de type AAV contenant le vecteur lentiviral à triplex.

Un vecteur ainsi défini est transcomplémenté par un ou plusieurs vecteurs additionnels apportant les séquences codant pour les polypeptides de structure du génome d'un rétrovirus choisi, en particulier d'un lentivirus, ou les polypeptides de structure d'un rétrotransposon.

A cet égard, le vecteur obtenu selon l'invention peut être transcomplémenté par l'apport de séquences codant pour les polypeptides GAG, POL et ENV, ou pour une partie de ces polypeptides suffisante pour permettre la formation de particules rétrovirales destinées à vectoriser le vecteur recombinant dépourvu des gènes viraux et comportant le transgène dont on recherche l'expression.

Un vecteur selon l'invention peut être caractérisé par le fait que le transgène ou la séquence d'intérêt est contenu(e) dans une cassette d'expression comprenant des signaux régulateurs de transcription et d'expression.

De façon générale, le(s) vecteur(s) utilisés pour la trans-complémentation en protéines rétrovirales ou rétroviraloïdes, sont dépourvus de signaux d'encapsidation.

A ce sujet, on citera les vecteurs préparés selon les techniques de Goldman et al (1997) utilisables pour la transcomplémentation d'un vecteur recombinant selon l'invention.
La demande décrit également des particules vecteur rétrovirales recombinantes comprenant :
a) un polypeptide gag correspondant à des nucléoprotéines d'un lentivirus ou à des polypeptides dérivés fonctionnels (polypeptides GAG),
b) un polypeptide pol constitué par les protéines RT, PRO, IN d'un lentivirus ou un polypeptide dérivé fonctionnel (polypeptide POL),
c) un polypeptide d'enveloppe ou des polypeptides dérivés fonctionnels (polypeptides ENV),
d) une séquence de nucléotides recombinant comprenant une séquence de nucléotides déterminée (transgène ou une séquence d'intérêt) placée sous le contrôle de signaux régulateurs de transcription et d'expression, une séquence contenant des signaux régulateurs de rétrotranscription, d'expression et d'encapsidation d'origine rétrovirale ou rétrovirale-like et un polynucléotide comportant une région cis-active d'initiation centrale (cPPT) et une région cis-active de terminaison (CTS), ces régions étant d'origine rétrovirale ou rétrovirale-like et étant insérées dans une orientation fonctionnelle avec les susdits signaux régulateurs d'origine rétrovirale ou rétrovirale-like.

La demande décrit aussi des particules vecteur rétrovirales recombinantes comprenant :
a) une séquence de nucléotides dite séquence gag codant pour les nucléoprotéines d'un lentivirus ou pour des polypeptides dérivés fonctionnels (polypeptides GAG),
b) une séquence de nucléotides dite séquence pol codant pour les protéines RT, PRO, IN et RN d'un lentivirus ou pour un polypeptide dérivé fonctionnel (polypeptide POL),
c) des signaux régulateurs de transcription et d'expression des séquences gag et pol,
d) une séquence de nucléotides dite séquence env codant pour des polypeptides d'enveloppe ou pour des polypeptides dérivés fonctionnels (polypeptides ENV), la séquence env étant placée sous le contrôle de signaux régulateurs de transcription et d'expression,
e) une séquence de nucléotides recombinante comprenant une séquence de nucléotides déterminée (transgène), placée sous le contrôle dé signaux régulateurs de transcription et d'expression, une séquence contenant des signaux régulateurs de rétrotranscription, d'expression et d'encapsidation d'origine rétrovirale ou rétrovirale-like et un polynucléotide comportant une région cis-active d'initiation centrale (cPPT) et une région cis-active de terminaison (CTS),, ces régions étant d'origine rétrovirale ou rétrovirale-like, ces régions étant insérées dans une orientation fonctionnelle avec les signaux régulateurs d'origine rétrovirale ou rétrovirale-like.

L'invention a pour objet l'utilisation d'une séquence de nucléotides comprenant un polynucléotide comportant une région cis-active d'initiation centrale (cPPT) et une région cis-active de terminaison (CTS), d'origine lentivirale ou lentiviraloïde chacune de ces deux régions encadrant un enchaînement de nucléotides interne, les susdites régions cis-actives cPPT et CTS étant insérées au sein de la susdite séquence de nucléotides, dans une orientation fonctionnelle avec des signaux régulateurs de rétrotranscription d'origine lentivirale ou lentiviraloïde.

Les polypeptides GAG et POL sont les polypeptides de nucléoprotéine issues de précurseurs clivés par la protéase virale. Les polypeptides POL comprennent la réverse transcriptase (RT), la protéase (PRO), l'intégrase (IN) et la Rnase H (RN) du rétrovirus. Le cas échéant, d'autres protéines du rétrovirus sont également utilisées pour la construction des particules vecteur. On note que les termes protéines ou polypeptides utilisés couvrent les formes non glycosylées ou les formes glycosylées des polypeptides en question.

Les séquences gag, pol et env utilisées pour la construction des particules vecteur rétrovirales peuvent être, le cas échéant, modifiées par mutation, par exemple par mutation ponctuelle ou par délétion ou insertion d'un ou plusieurs nucléotides, ou issues de chimères recombinantes provenant de différents rétrovirus par exemple entre HIV1 et HIV2 ou HIV1 et CAEV (Caprine Arthritis Encephalitis Virus), dès lors qu'elles permettent la production de polypeptides fonctionnels pour la production de particules virales capables de vectoriser le transgène à exprimer. En particulier, on aura recours à des séquences mutées pour accroître la sécurité du rétrovirus ainsi produit.

Avantageusement, le vecteur recombinant obtenu selon l'invention ou les particules vecteur recombinantes sont tels que le transgène est sous le contrôle de signaux régulateurs de transcription et d'expression d'origine non rétrovirale. Un promoteur susceptible d'être utilisé pour contrôler l'expression du transgène, est par exemple le promoteur du CMV, le promoteur PGK ou EF1α décrit dans Tripathy, SK et al. (PNAS 1994, 91, p 11557-11561).

Selon une variante de réalisation de l'invention, le transgène peut toutefois être placé sous le contrôle des signaux régulateurs identifiés précédemment comme étant d'origine lentivirale ou lentiviraloïde en particulier sous le contrôle de la séquence LTR.

Un lentivirus utilisé pour dériver la construction lentivirale selon l'invention peut être choisi parmi les rétrovirus HIV, par exemple HIV-1, HIV-2 ou tout isolat différent de ces deux types, ou par exemple parmi les virus CAEV (Caprine Arthritis Encephalitis Virus), EIAV (Equine Infectious Anemia Virus), VISNA, SIV (Simian Immunodeficiency Virus), ou FIV (Feline Immunodeficiency Virus).

Un vecteur particulièrement avantageux selon l'invention est le vecteur caractérisé en ce que le polynucléotide est une séquence d'ADN comprenant la région cis-active d'initiation centrale (cPPT) et la région de terminaison (CTS) du génome d'un rétrovirus HIV-1, ou de tout autre lentivirus.

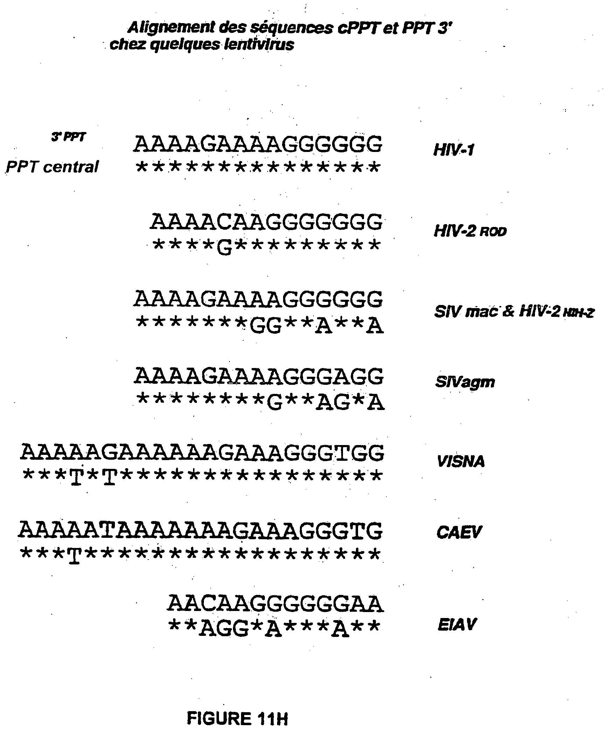

La séquence PPT centrale ou cPPT est une séquence relativement conservée au sein des lentivirus et est identifiée par la présence de nombreux résidus purines dont certaines sont montrées sur la figure 11H. Des mutations, même ponctuelles, au sein d'une de ces régions, peuvent abolir leur caractère fonctionnel lié à la formation des structures d'ADN triplex.

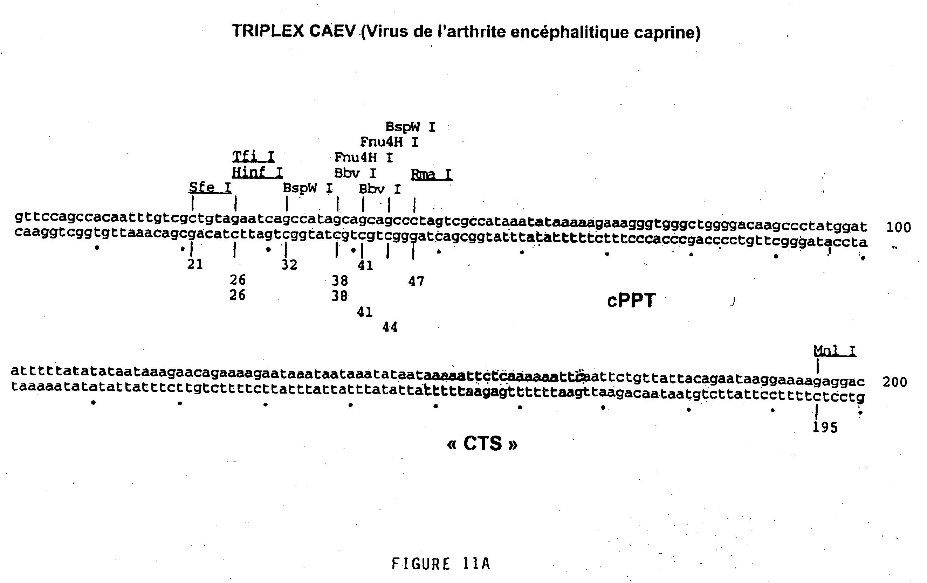

L'identification des séquences cPPT est facilitée par le fait qu'il s'agit de répétition au centre du génome chez les lentivirus, d'une séquence polypurine située à la bordure amont (5') du LTR 3' chez tous les rétrovirus. Cette séquence cPPT peut être une répétition exacte, comme dans le cas du virus HIV-1, ou légèrement modifiée chez d'autres lentivirus (figure 11 H). La séquence de terminaison centrale CTS est caractérisée dans le cas du virus HIV-1 (Chameau et al, 1994). Elle est située à une centaine de nucléotides en aval de la séquence cPPT. On trouve chez les autres lentivirus des séquences CTS candidates là encore à une centaine de nucléotides (de 80 à 120 nucléotides) en aval de la séquence cPPT. La position probable de la séquence CTS est indiquée chez quelques lentivirus sur les figures 11A à 11E.

La séquence CTS du lentivirus EIAV a récemment été caractérisée (Scott R. Stetor et al., Biochemistry 1999, 38, P 3656-67). Selon ces auteurs, chez EIAV, les séquences cPPT et CTS sont respectivement 5' AAC AAA GGG AGG GA 3' et 5' AAA AAA TTT TGT TTT TAC AAA ATC 3'.

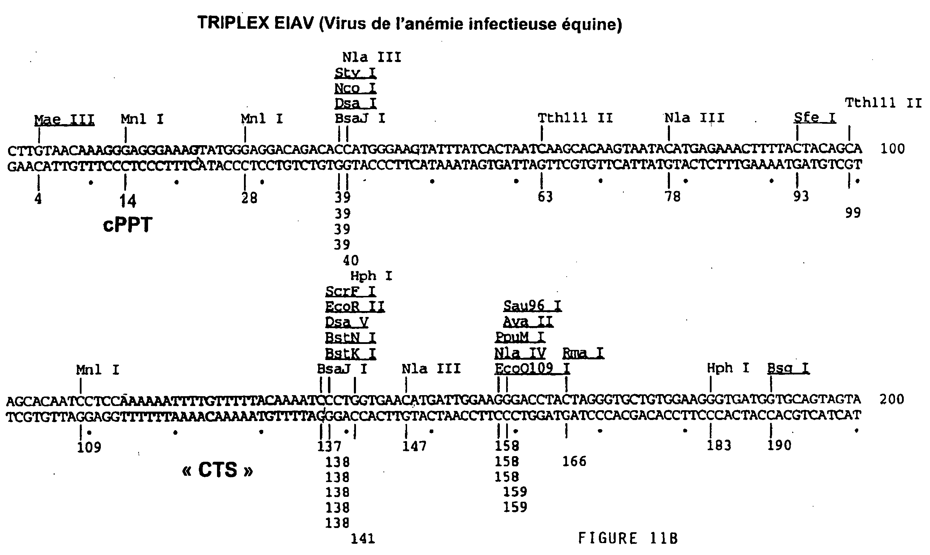

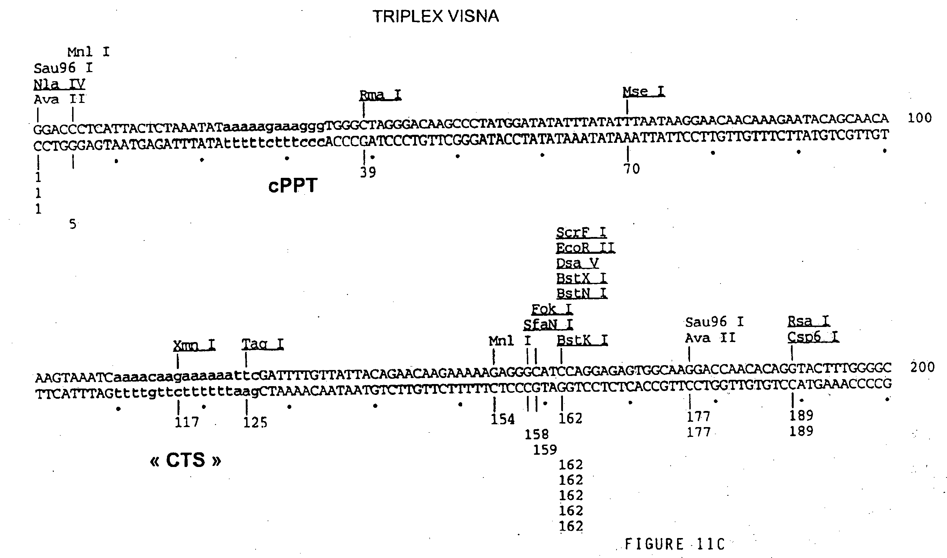

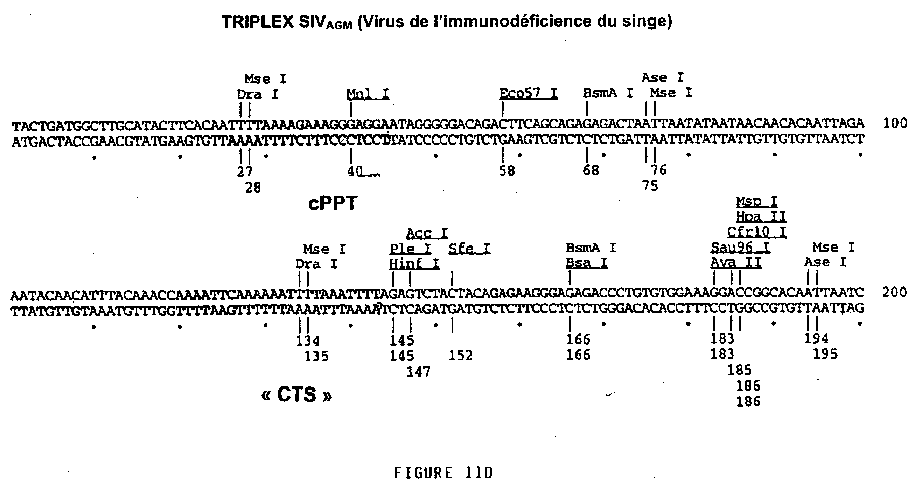

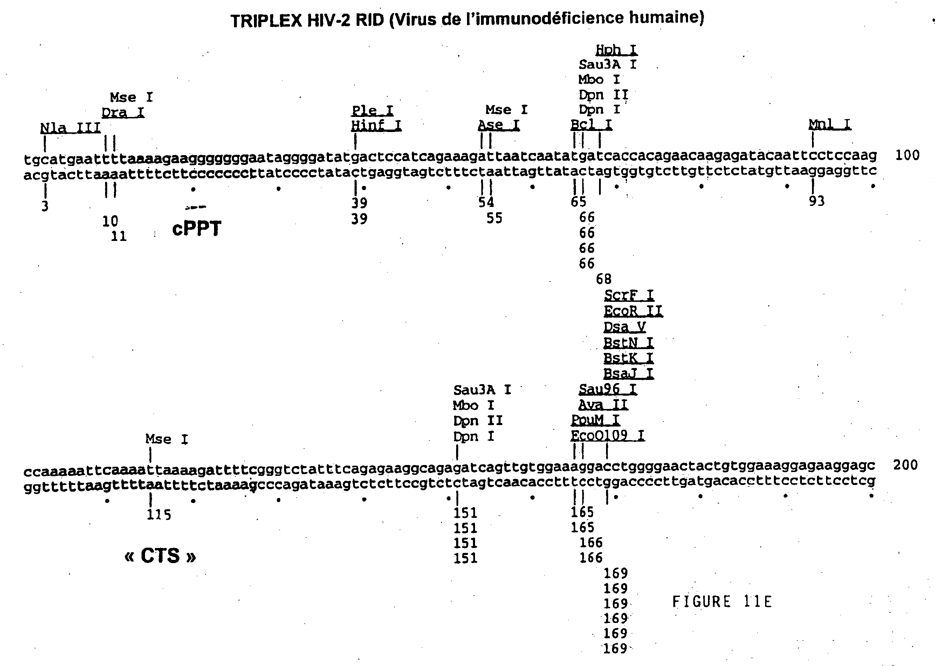

Parmi les polynucléotides préférés utilisables dans le cadre de l'invention, on cite par exemple les séquences représentées à la figure 11, et plus précisément les séquences comprises entre les deux régions cPPT et CTS, incluant les séquences desdites régions.

Le cas échéant, la séquence de nucléotides comprenant le cPPT, le polynucléotide interne (c'est à dire liant le cPPT jusqu'à la séquence CTS) et la séquence CTS, peut être mutée ponctuellement, ou mutée par délétion ou insertion de nucléotides. A titre d'exemple, des mutations ponctuelles ont été réalisées dans la séquence cPPT de HIV1 et ont montré qu'il y avait maintien d'une infectivité résiduelle dans les cellules (Charneau et al, J. Virol. 1992, 66, p. 2814-2820).

L'invention couvre l'utilisation de toute séquence mutée pour cPPT ou CTS ayant au moins 60 % d'identité avec la séquence nucléotidique cis active homologue naturelle d'où elle est issue. En cas de séquences actives en cis chimères, le pourcentage s'applique à chaque séquence nucléotidique mutée de la chimère.

Des modifications de la séquence nucléotidique des régions PPT ou cPPT ou CTS peuvent être introduites pour construire l'ADN triplex selon l'invention. Ces modifications peuvent atteindre jusqu'à 40 % de la séquence naturelle.

L'identité des séquence nucléotidiques variantes par rapport aux séquences dites naturelles, se calcule strictement par rapport au cPPT ou au CTS individuellement et non par rapport à la séquence nucléotidique de l'ADN triplex complet.

La région comprise entre le cPPT et le CTS, est constituée par un polynucléotide qui peut être soit celui trouvé dans le génome lentivirale d'origine entre le CTS et le PPT soit être différent de celui-ci à condition que l'ADN triplex conserve ses propriétés dans l'import nucléaire du polynucléotide permettant d'amener la séquence nucléotidique d'intérêt à l'intérieur du noyau.

Le polynucléotide décrit dans la demande peut être introduit dans un vecteur réplicatif ou non réplicatif. Dans le cas d'un vecteur rétroviral, il s'agit d'un vecteur de type non réplicatif.

Pour la préparation de quantités importantes de particules vecteur lentiviral, il est possible d'utiliser des vecteurs de type adénoviral dans lesquels a été introduit le polynucléotide correspondant au génome lentiviral qui contient les séquences ADN triplex et celles des gènes gag, pol, et env.

Ces vecteurs adénoviraux peuvent éventuellement être rendus réplicatifs par l'introduction d'une séquence origine de réplication.

Dans la figure 11G, les séquences cPPT et CTS du HIV-1 sont encadrées.

En tout état de cause, on utilisera des séquences mutées qui conservent la capacité à former un triplex d'ADN lors de la rétrotranscription du génome dans la cellule cible.

Un vecteur recombinant selon un mode de réalisation particulier peut, donc comprendre, tout ou partie des séquences LTR rétrovirales ou de rétrotranscription, les sites rétroviraux PBS et PPT 3'-terminal, la séquence rétrovirale nécessaire à l'encapsidation du génome vecteur dans la particule vecteur. La séquence LTR peut être en partie délétée, notamment dans la région U3.

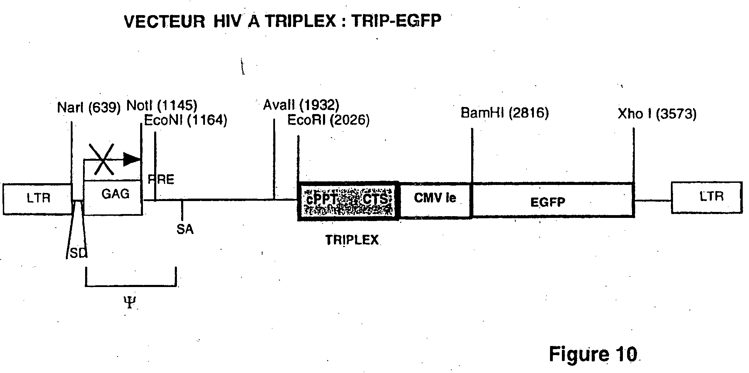

Un vecteur particulier est le plasmide pTRIP.EGFP, déposé à la CNCM (Collection Nationale de Cultures de Microorganismes de l'Institut Pasteur, France) le 15 avril 1998, sous le numéro I-2005. La carte de restriction de ce vecteur est représentée à la figure 10.

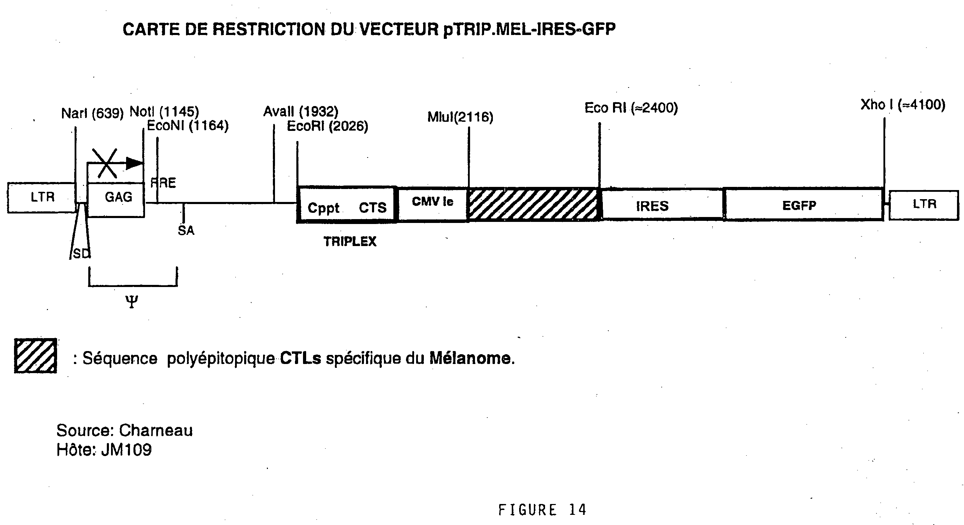

Un autre vecteur est le plasmide pTRIP.MEL-IRES-GFP déposé à la CNCM le 20 Avril 1999 sous le n° I-2185. Ce vecteur est le plasmide pTRIP.MEL-IRES-GFP représenté à la figure 14.

Un vecteur recombinant particulier décrit dans la demande est caractérisé en ce que les séquences gag, pol et env sont également dérivées des séquences d'un lentivirus, en particulier d'un rétrovirus HIV, en particulier HIV-1 ou HIV-2.

Selon un autre mode de réalisation de l'invention, les séquences gag et pol sont dérivées d'un lentivirus HIV et la séquence env est dérivée d'un rétrovirus distinct de HIV ou d'un virus, par exemple le virus de la somatite vésiculeuse (VSV).

De façon générale et en fonction de l'expression du transgène que l'on recherche, on choisira d'avoir recours à une séquence env codant pour des polypeptides env amphotropes par rapport à l'hôte dans lequel on souhaite exprimer le transgène, ou au contraire on choisira des séquences env codant pour des polypeptides env écotropes. Le tropisme de la séquence env peut être un tropisme spécifiquement humain.

La demande décrit aussi des particules vecteur recombinantes comprenant une séquence de nucléotides recombinante comprenant une séquence de nucléotides déterminée (transgène ou séquence d'intérêt), placée sous le contrôle de signaux régulateurs de transcription et d'expression, des signaux régulateurs de rétrotranscription, d'expression et d'encapsidation et un polynucléotide comportant une région cis-active d'initiation centrale (cPPT) et une région cis-active de terminaison (CTS).

Elle décrit également des particules vecteur recombinantes comprenant une séquence de nucléotides recombinante contenant une séquence de nucléotides déterminée (transgène), placée sous le contrôle de signaux régulateurs de transcription et d'expression, des signaux régulateurs de rétrotranscription, d'expression et d'encapsidation de rétrotransposon et un polynucléotide comportant une région cis-active d'initiation centrale (cPPT) et une région cis-active de terminaison (CTS), ces régions étant dérivées d'un rétrotransposon et insérées dans une orientation fonctionnelle avec les signaux régulateurs de rétrotransposon.

De plus, la demande décrit aussi des particules vecteur recombinantes comprenant :
a) un polypeptide GAG correspondant aux nucléoprotéines d'un rétrotransposon ou à des polypeptides dérivés fonctionnels,
b) un polypeptide POL correspondant aux protéines RT, PRO, IN d'un rétrotransposon ou à un polypeptide dérivé fonctionnel,
c) des signaux régulateurs de transcription et d'expression des séquences gag et pol,
d) une séquence de nucléotides recombinante comprenant une séquence de nucléotides déterminée (transgène), placée sous le contrôle de signaux régulateurs de transcription et d'expression, une séquence contenant des signaux régulateurs de rétrotranscription, d'expression et d'encapsidation de rétrotransposon et un polynucléotide comportant une région cis-active d'initiation centrale (cPPT) et une région cis-active de terminaison (CTS), ces régions étant dérivées d'un rétrotransposon et insérées dans une orientation fonctionnelle avec les signaux régulateurs de rétrotransposon.

De plus, la demande décrit aussi des particules vecteur recombinantes résultant de l'expression de :
a) une séquence de nucléotides dite séquence gag codant pour les nucléoprotéines d'un rétrotransposon ou pour des polypeptides dérivés fonctionnels (polypeptides GAG),
b) une séquence de nucléotides dite séquence pol codant pour la protéine RT, PRO et IN d'un rétrotransposon ou pour un polypeptide dérivés fonctionnel (polypeptide POL),
c) des signaux régulateurs de transcription et d'expression des séquences gag et pol, lesdites particules comprenant une séquence de nucléotides recombinante comprenant- une séquence de nucléotides déterminée (transgène), placée sous le contrôle de signaux régulateurs de transcription et d'expression, une séquence contenant des signaux régulateurs de rétrotranscription, d'expression et d'encapsidation de rétrotransposon et un polynucléotide comportant une région cis-active d'initiation centrale (cPPT) et une région cis-active de terminaison (CTS), ces régions étant dérivées d'un rétrotransposon et insérées dans une orientation fonctionnelle avec les signaux régulateurs de rétrotransposon.

La demande décrit aussi des particules rétrovirales-like recombinantes comprenant :
a) un polynucléotide comportant une région cis-active d'initiation centrale (cPPT) et une région cis-active de terminaison (CTS), ces régions étant dérivées d'un rétrotransposon et insérées dans une orientation fonctionnelle avec les signaux régulateurs de rétrotransposon,
b) un polypeptide correspondant aux nucléoprotéines d'un rétrotransposon ou à des polypeptides dérivés fonctionnels (polypeptides GAG),
c) un polypeptide pol correspondant à des protéines RT, PRO, IN d'un rétrotransposon ou un polypeptide dérivé fonctionnel (polypeptide POL),
d) un polypeptide d'enveloppe virale,
e) une séquence de nucléotides recombinante comprenant une séquence de nucléotides déterminée (transgène ou séquence d'intérêt), placée sous le contrôle de signaux régulateurs de transcription et d'expression, des signaux régulateurs de rétrotranscription, d'expression et d'encapsidation de rétrotransposon.

La demande décrit par exemple un vecteur recombinant tel que défini précédemment, dans lequel les signaux régulateurs de rétrotranscription, d'expression et d'encapsidation et le polynucléotide comprenant les régions cPPT et CTS, sont dérivés d'un rétrotransposon, par exemple d'un rétrotransposon de levure.

De façon générale, les signaux régulateurs de transcription et d'expression du transgène ou des séquences codant pour les polypeptides de structure des particules vecteur, lorsqu'ils ne sont pas d'origine rétrovirale ou rétroviraloïde, sont avantageusement des signaux inductibles ou conditionnels, susceptibles de conduire à une expression tissulaire spécifique.

Sont également décrites, des cellules recombinantes caractérisées en ce qu'elles sont recombinées avec un vecteur selon l'une des définitions précédentes. La recombinaison peut être effectuée par tout moyen disponible; et en particulier par transfection ou par infection, notamment par transfection ou par transduction par un vecteur.

Les cellules ainsi peuvent être transfectées de façon transitoire, ou au contraire de façon stable. Il peut s'agir de cellules d'encapsidation ou de cellules cibles, en particulier de cellules dans lesquelles on recherche un effet thérapeutique par l'expression du transgène.

De façon tout à fait intéressante, des cellules recombinantes capables d'exprimer le transgène grâce à la transduction à l'aide d'un vecteur de l'invention, sont des cellules eucaryotes différenciées non mitotiques.

La divulgation permet cependant également la préparation de cellules recombinantes eucaryotes primaires non mitotiques, ou encore de cellules mitotiques.

A titre d'exemple, on citera les cellules de poumon, les cellules du cerveau, épithéliales, astrocytes, microglie, oligodendrocytes et neurones, musculaires, hépatiques, dendritiques, neuronales, les cellules de la moelle osseuse, les macrophages, les fibroblastes, les lymphocytes, les cellules hématopoïétiques.

La demande décrit donc des compositions à visée thérapeutique, caractérisées en ce qu'elles comprennent un vecteur tel que précédemment décrit, ou une cellule recombinante définie selon les indications précédentes.

La demande décrit également une composition immunogène comprenant un vecteur tel que décrit précédemment ou des cellules recombinantes définies ci-dessus, ladite composition étant susceptible de conduire à une réponse immunitaire, cellulaire ou humorale chez un hôte déterminé.

La demande décrit donc un polynucléotide tel que défini précédemment, comprenant des régions cPPT et CTS lentivirales ou lentiviraloïdes et donne accès à son utilisation pour l'import nucléaire, notamment ex vivo dans des cellules déterminées, d'une séquence de nucléotides (transgène).

En outre, la divulgation met à disposition un polynucléotide tel que défini ci-dessus associé à une séquence nucléotidique d'intérêt ou à un transgène.

Enfin, la demande décrit l'utilisation d'un polynucléotide comportant une région cis-active d'initiation centrale (cPPT) et une région cis-active de terminaison (CTS), ces régions étant d'origine lentivirale ou lentiviraloïde pour la transfection ou la transduction de cellules eucaryotes avec un transgène ou un polynucléotide d'intérêt.

Elle décrit également l'utilisation d'un vecteur recombinant ou d'un polynucléotide de l'invention pour la transduction in vivo.

D'autres caractéristiques et avantages de l'invention apparaissent dans les exemples et dans les figures qui suivent

### LEGENDES DES FIGURES

**FIG. 1** **: Rétrotranscription des Lentivirus**
   La rétrotranscription des génomes lentiviraux diffère de celle des rétrovirus oncogènes par la synthèse du brin+ en deux moitiés distinctes. Un segment aval est initié au niveau d'une copie centrale du polypurine tract (cPPT), caractéristique des génomes lentiviraux. La synthèse du brin+ amont se termine après un déplacement de brin discret au centre du génome. Le blocage du déplacement de brin par la reverse transcriptase est gouverné par une séquence cis-active du génome HIV : le CTS (Central Germination Sequence). Le produit final de la rétrotranscription des lentivirus est un ADN linéaire portant une structure ADN centrale à trois brins (triplex central) sur une longueur d'une centaine de nucléotides.

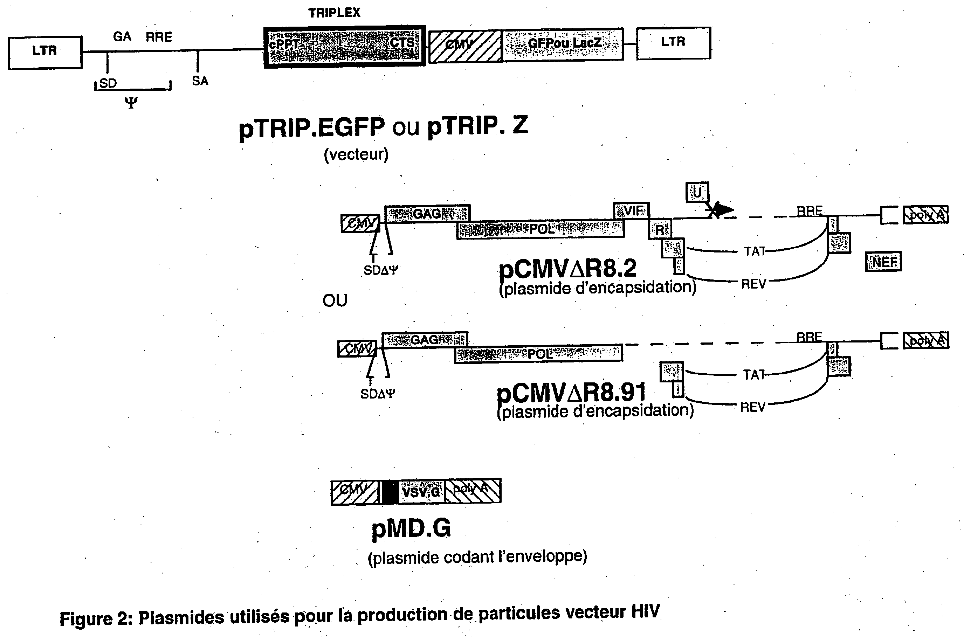

**FIG. 2** **: Plasmides utilisés pour la production des particules vecteur HIV**
   Les particules vecteur sont produites par cotransfection, de trois plasmides : le plasmide vecteur comprenant (pTRIP) ou non (pHR) les séquences cis-actives responsables de la formation de triplex, un plasmide d'encapsidation fournissant en trans les protéines structurales et les enzymes de la particule (pCMVΔR8.2 ou pCMVΔR8.91, Naldini et al, 1996 et Zufferey et al, 1997), et un plasmide d'expression de l'enveloppe du virus VSV (VSV=G).
   Seules les parties pertinentes des plasmides cotransfectés dans des cellules Hela sont présentes (Naldini et al PNAS oct 1996, Zufferey et al Nature Biotech. 1997).
   Les plasmides d'encapsidation pCMVΔR8.2 ou pCMVΔ8.91 permettent l'expression des protéines issues de gag et pol.
   pMD.G code pour l'envelope hétérologue VSV. Les plasmides vecteur pHR-TRIP sont dérivés du plasmide pHR'CMVIacZ (Naldini et al) une séquence triplex sauvage ou mutante a été insérée et le gène rapporteur lacZ changé ou non en EGFP.

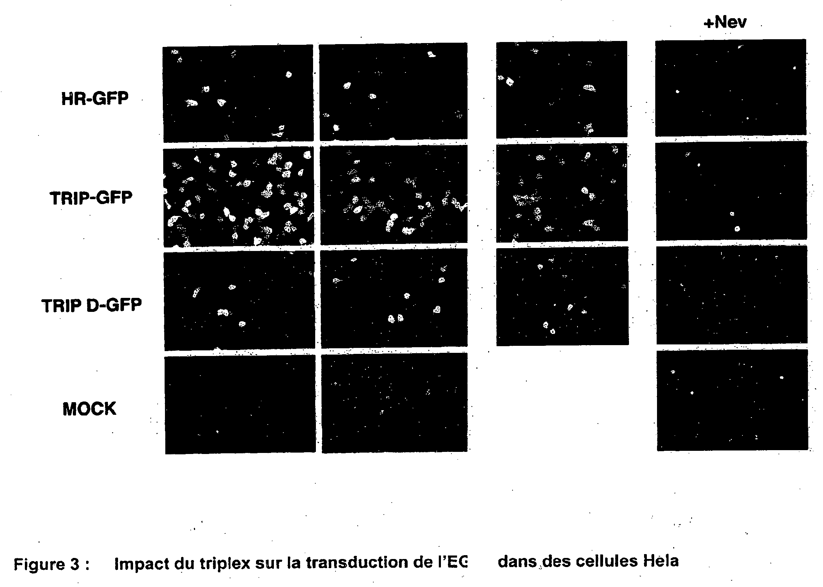

**FIG. 3** **: impact du triplex sur la transduction de l'EGFP dans des cellules Hela**
   Des cellules Hela, cultivées en Labtek à 8 chambres, sont transduites par différents vecteurs exprimant la protéine autofluorescente EGFP. Les infections sont normalisées selon la quantité de protéine de capside (kit ELISA P24 Dupont) à 2 ng de P24 par inoculum. A 48 heures post-infection, les cellules sont fixées au PBS PFA 1 %, montées en mowiol, puis observées au microscope à fluorescence. Trois champs indépendants sont montrés pour le vecteur d'origine sans triplex (HR.EGFP, en haut), pour le vecteur avec le triplex (TRIP.EGFP, milieu) ou pour un vecteur contenant une séquence triplex mutée, non fonctionnelle (TRIP D.EGFP, en bas). A droite sont montrées les différentes transductions en présence de nevirapine, un inhibiteur de la rétrotranscriptase du HIV-1.

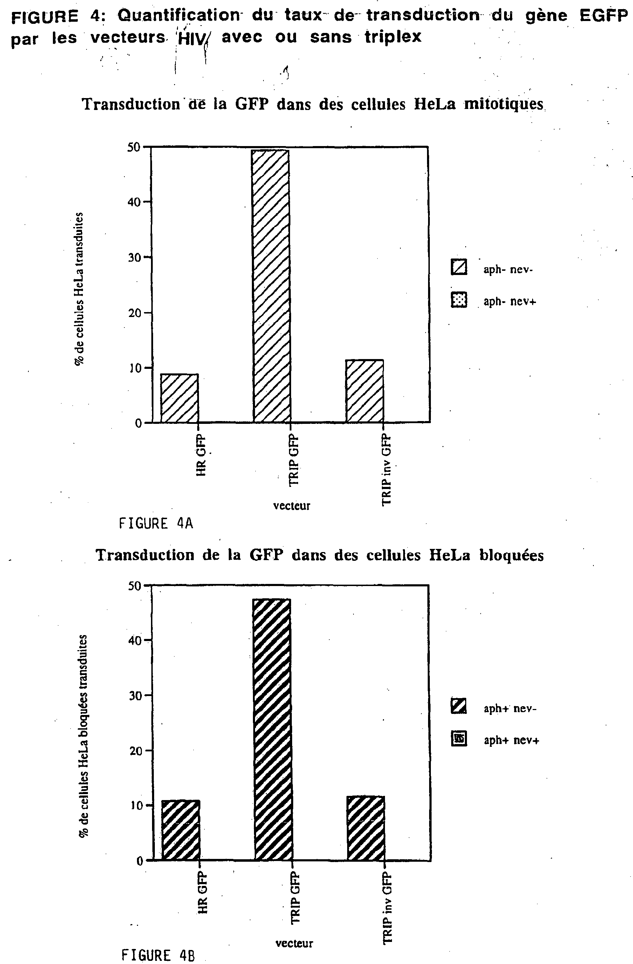

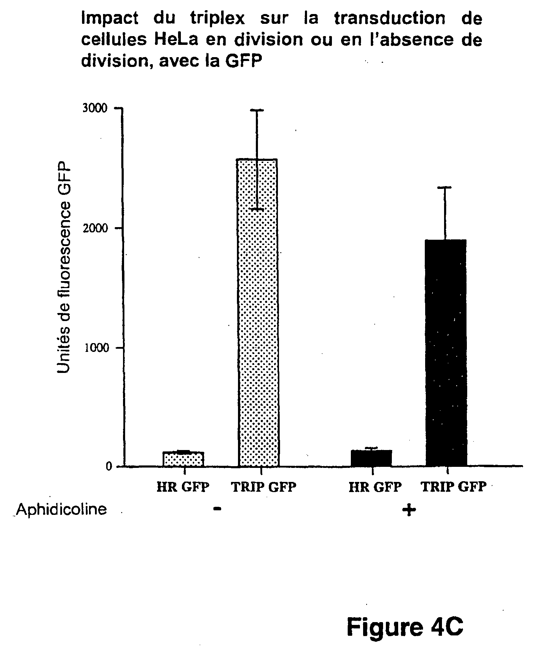

**FIG. 4** **: Quantification du taux de transduction du gène EGFP par les vecteurs HIV avec ou sans triplex**
   Des cellules Hela transduites par 2 ng P24 des vecteurs EGFP avec ou sans triplex sont trypsinnées à 48 heures post-infection. Les pourcentages de cellules positives pour l'expression de l'EGFP sont calculés par cytométrie en flux (canal FITC). Dans tous les cas, la transduction est inhibée en présence de nevirapine, un inhibiteur de la rétrotranscriptase du HIV-1. Dans la figure 4C, on observe la présence de l'ADN triplex dans le vecteur stimulé par la transduction de GFP (ou autre gène d'intérêt) dans les cellules en mitose ou non mitotiques. Cette transduction est multipliée par un facteur 20 par rapport aux résultats obtenus avec des vecteurs sans séquence triplex (par exemple cf. Naldini et al, Science, 1996).

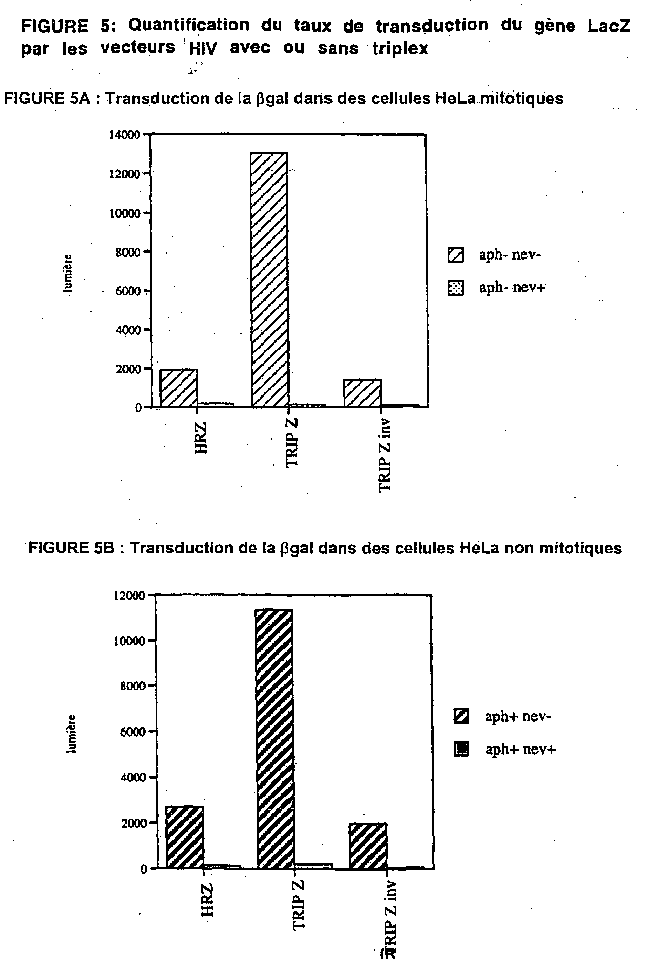

**FIG. 5** **: Quantification du taux de transduction du gène LacZ, par les vecteurs HIV avec ou sans triplex**
   L'impact du triplex sur la transduction est calculé par infection de cellules Hela, cultivées en plaques 96 puits, par différents vecteurs exprimant le gène rapporteur LacZ. A 48 heures, post-infection, les plaques de cultures sont lysées, et l'activité beta-Galactosidase est mesurée selon un kit de réaction luminescente (Boehringer). Chaque transduction est réalisée en triplicate avec un innoculum normalisé à 2 ng de P24.
   Panneau supérieur : Cellules Hela en prolifération.
   Panneau inférieur : Cellules Hela bloquées en cycle par l'aphidicoline.
   La transduction du gène LacZ est multipliée par un facteur 6 avec un vecteur contenant une séquence triplex par rapport à un vecteur sans séquence triplex.
**Fig. 6a** **et b :Impact du triplex sur la transduction *ex vivo* du gène EGFP dans des cellules spinales primaires de rat**
   Des cellules d'explants primaires de moelle épinière de rat sont infectées par 300 ng de P24 pour chaque vecteur avec et sans triplex, et observées en microscopie à fluorescence comme précédemment.
**FIG. 7** : **Impact du triplex sur la transduction in vivo du gène EGFP et du gène de la luciférase dans le cerveau de rat**

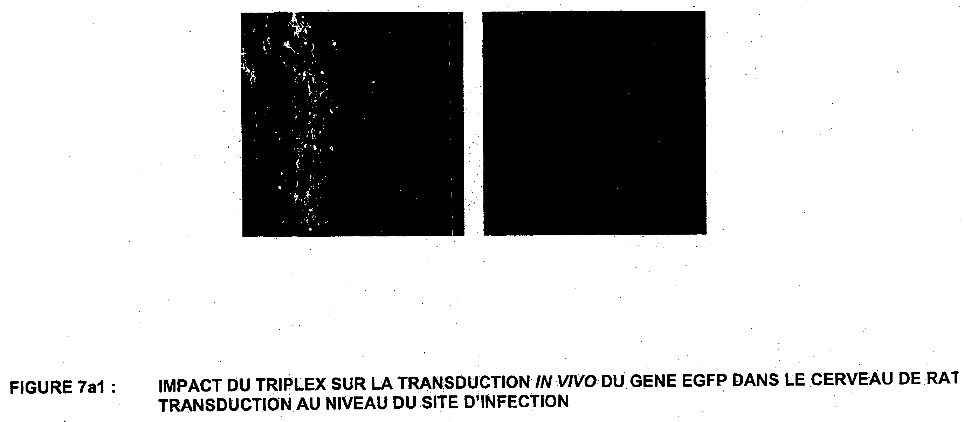

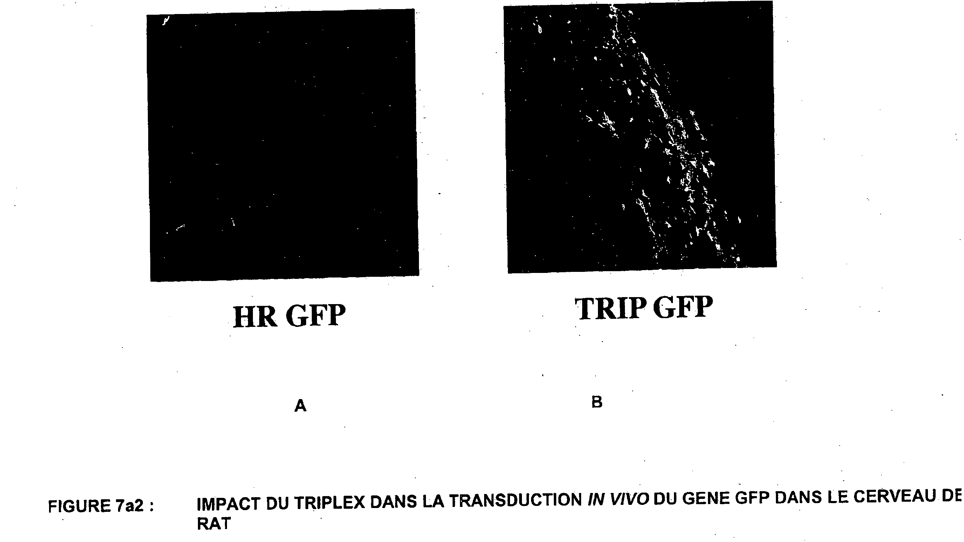

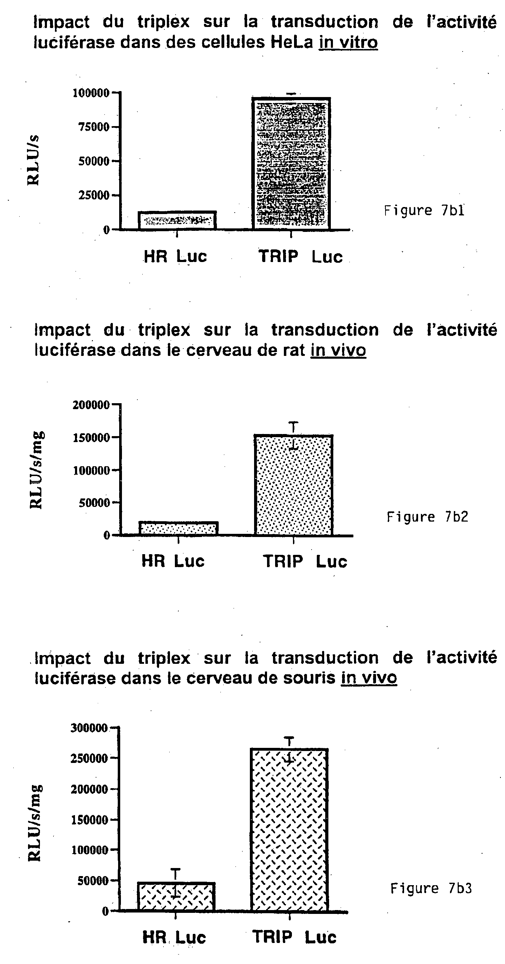

   FIG 7-a-1: Transduction au niveau du site d'injection
      Le transfert du gène EGFP est réalisé par injection directe dans le striatum du cerveau de rat de 2 microlitres de vecteur correspondant à 50 ng de P24.
      L'observation des coupes en microscopie à fluorescence montre une transduction importante de EGFP en présence du triplex (panneau gauche), et très faible sans (panneau droit).
   FIG 7-a-2: autre section représentative de l'expérience décrite ci-dessus.
   FIG 7-b: **Quantification de l'impact du triplex sur la transduction in vivo dans le cerveau.**
   FIG 7-b-1: Impact du triplex sur la transduction du gène codant pour la luciférase dans les cellules Hela in vitro. Le graphique montre la production de luciférase quantifiée par mesure de luminescence (kit Promega ®). La présence du triplex dans le vecteur permet d'obtenir une augmentation de la transduction du gène de la luciférase, d'un facteur 8.
   FIG 7-b-2: In vivo, quantification de l'activité luciférase dans des cerveaux de rat après injection de vecteurs codant pour la luciférase, avec ou sans triplex. La présence de triplex stimule la transduction de la luciférase d'un facteur 8.
   FIG 7-b-3: Même expérience que 7-b-2 mais réalisée chez la souris.

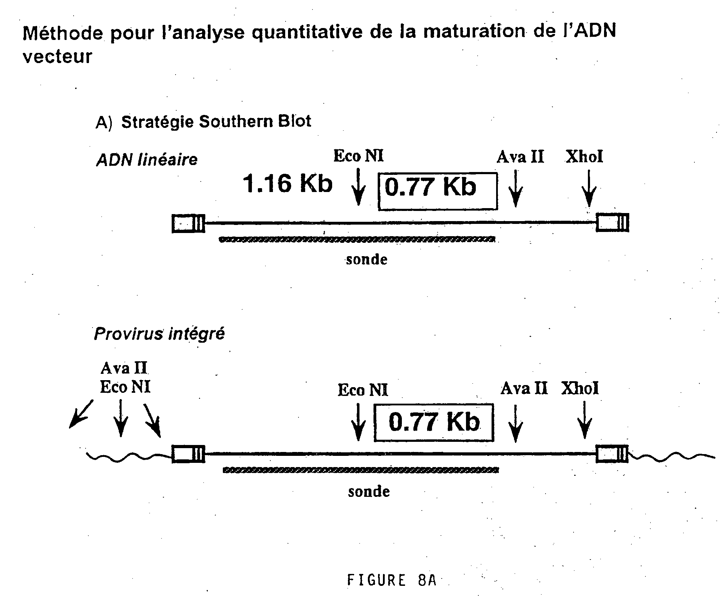

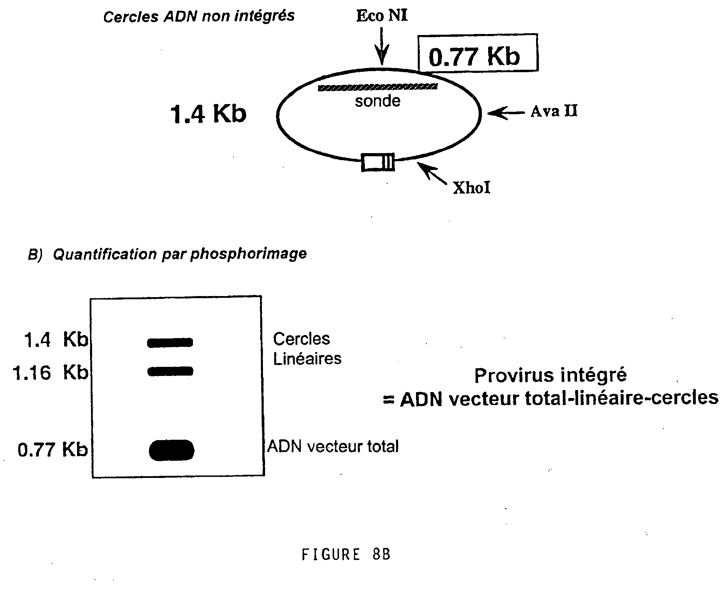

**FIG. 8** : **Stratégie d'analyse du taux d'import nucléaire de l'ADN vecteur.**
   Un test quantitatif permettant de suivre la cinétique de rétrotranscription, l'import nucléaire et l'intégration ou la circularisation de l'ADN vecteur dans les cellules transduites a été mis au point. Ce test remplace avantageusement la détection par amplification PCR des cercles à deux LTRs, marqueurs de l'import nucléaire de l'ADN viral dans le noyau de la cellule infectée (Bukrinsky et al, Nature 1993. 365, p. 666-669). L'ADN vecteur linéaire non intégré, les ADNs circulaires à un ou deux LTRs et l'ADN vecteur intégré sont détectés par Soutern blot et quantifiés au Phosphorimager suivant la stratégie de digestion de restriction suivante : L'ADN total des cellules transduites est digéré par EcoNI et Avall (deux sites uniques dans le génome vecteur), puis hybridé avec une sonde ADN générée par PCR chevauchant exactement le site EcoNI. Cette sonde réagit avec différents fragments : le fragment interne de 0.77 Kb, commun à toutes les formes ADN vecteur, et dont la quantification au phosphorimager indique la quantité totale d'ADN vecteur rétrotranscrit, un fragment distal de 1.16 Kb indiquant spécifiquement la quantité d'ADN linéaire non intégré. Après une digestion supplémentaire par l'enzyme Xhol, les cercles à un et deux LTRs apparaissent respectivement à 1.4 Kb et 2 Kb. La quantité d'ADN vecteur intégré est calculée en retranchant au signal correspondant à l'ADN total rétrotranscrit les signaux correspondant à l'ADN vecteur non intégré, ADN linéaire et cercles. Dans le cas d'un défaut d'import nucléaire, le profil attendu d'ADN vecteur dans les cellules transduites est une accumulation d'ADN linéaire non intégré. Au contraire, si l'ADN linéaire vecteur accède au compartiment nucléaire de la cellule, l'essentiel de l'ADN linéaire s'intègre dans la chromatine cellulaire et se circularise.

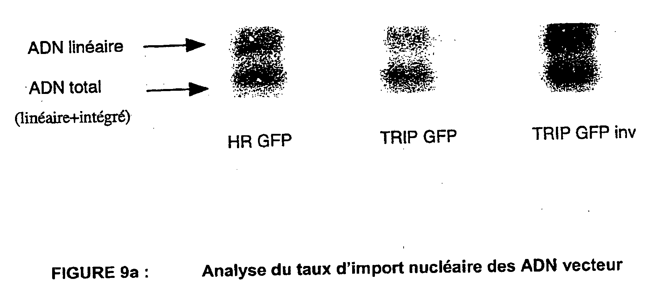

**FIG. 9a** **: Analyse du taux d'import nucléaire des ADNs vecteur.**
   L'analyse par southern blot, 48 heures post-transduction dans les cellules Hela, montre un défaut d'import nucléaire typique dans le cas du vecteur sans triplex (HR GFP) ou contenant la séquence triplex dans l'orientation inverse, non fonctionnelle (TRIP. GFP inv). Dans le cas de ces vecteurs, le signal correspondant à l'ADN linéaire non intégré est équivalent au signal ADN total, indiquant que l'essentiel de l'ADN vecteur reste sous forme linéaire au lieu de s'intégrer. Dans le cas du vecteur TRIP.GFP, l'intensité du signal correspondant à l'ADN linéaire est très inférieure au signal ADN total, indiquant qu'une fraction importante de l'ADN vecteur a été importée dans le noyau et s'y est intégrée.

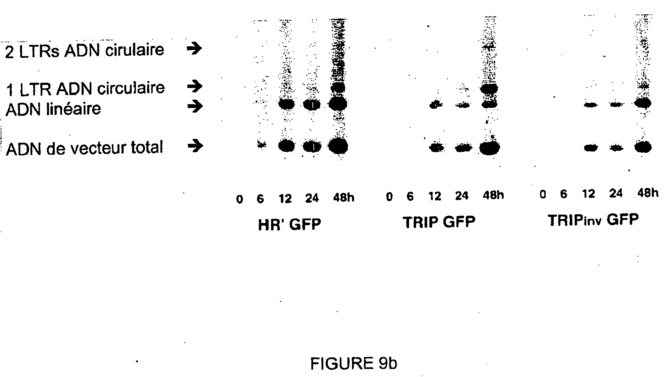

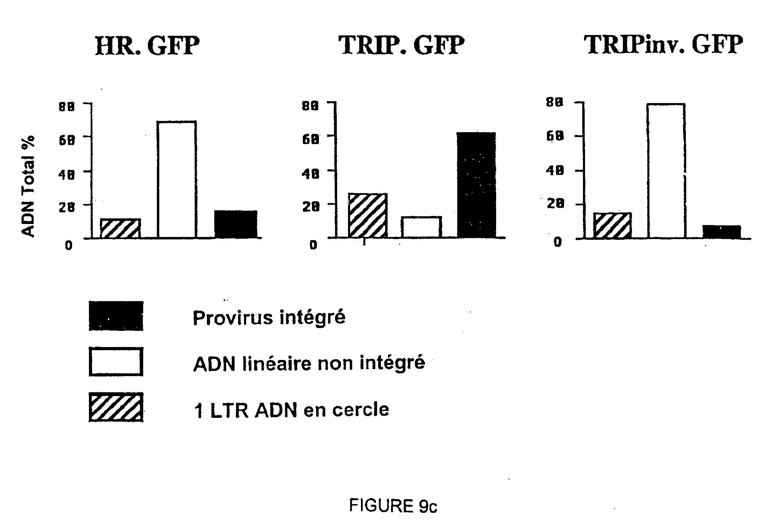

**Fig 9****-b:** Analyse cinétique du taux d'import nucléaire des ADNs vecteur avec triplex (TRIP-GFP) ou sans séquence triplex (HR-GFP, TRIPinv-GFP).
**Fig 9****-c:** Quantification de l'état de l'ADN vecteur dans les cellules transduites.
   La quantification au phosphorimager du Southern blot montré en figure 9b montre qu'à 48 heures post-transduction, la majorité de l'ADN des vecteurs sans triplex se trouve sous la forme d'ADN linéaire non intégré, peu d'ADN vecteur s'intègre ou se circularise. Les vecteurs sans triplex (HR-GFP et TRIPinv-GFP) montrent un défaut d'import nucléaire typique. A l'inverse, dans le cas du vecteur TRIP-GFP, plus de 60 % de l'ADN est intégré dans le génome de la cellule transduite et peu d'ADN vecteur subsiste sous forme d'ADN linéaire non intégré. L'introduction de la séquence triplex dans le vecteur a complémenté le défaut d'import nucléaire du vecteur HR-GFP jusqu'à un niveau sauvage. En effet, le profil d'ADN vecteur obtenu dans le cas du vecteur TRIP-GFP est comparable à celui d'un virus HIV-1 sauvage. Ce résultat démontre que la séquence triplex est le seul déterminant d'import nucléaire manquant dans la construction HR-GFP. Seule la forme intégrée de l'ADN vecteur est active.
**FIG. 10** **: Carte de restriction du vecteur pTRIP.EGFP.**
**FIGS. 11A - 11F** **: Séquence des polynucléotides comprenant les régions cPPT et CTS des virus CAEV, EIAV, VISNA, SIV_{AGM}, HIV-2_{ROD}, HIV-1_{LAI}.**
**La** **figure 11** **G** représente la séquence de l'ADN triplex du virus HIV1. Les régions actives en cis, cPPT et CTS, sont délimitées et imprimées en caractères gras majuscules.
**La** **figure 11H** représente l'alignement des séquences cPPT et PPT3' chez quelques lentivirus. La ligne du haut correspond à la séquence PPT 3' présente chez tous les rétrovirus en amont du LTR 3'. La ligne du bas correspond à la répétition interne de la séquence PPT dite cPPT chez les lentivirus.
**FIG. 12** **:**

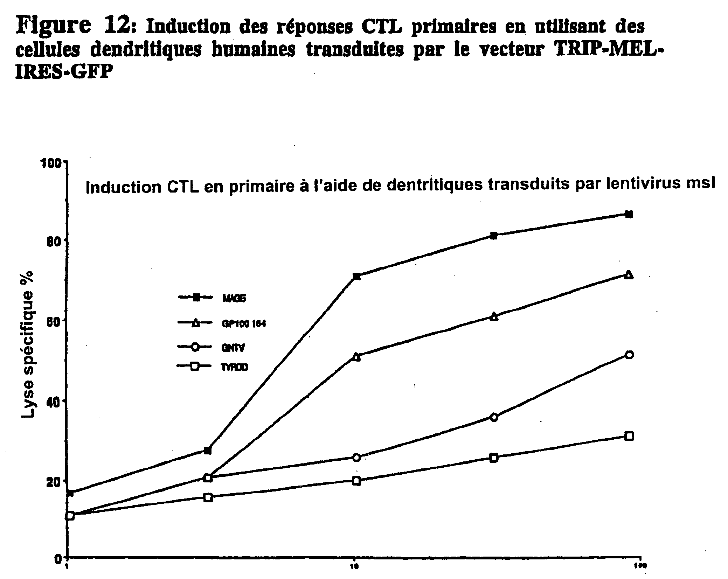

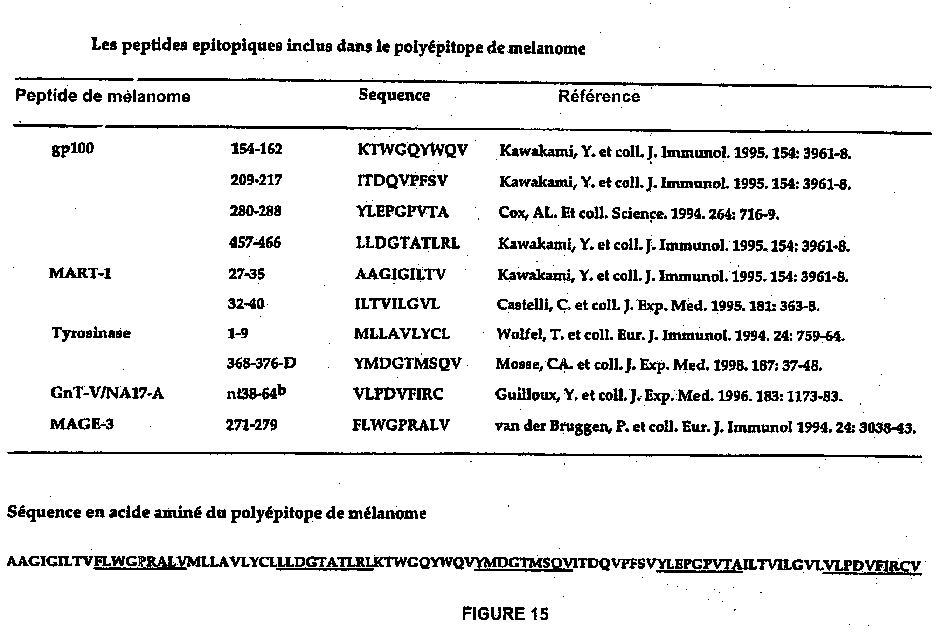

   La figure 12 représente la production de CTL in vitro à partir de cellules dendritiques humaines transduites par le vecteur triplex ayant comme genre d'intérêt un polyépitope CTL de mélanome constitué d'épitopes dont les séquences sont décrites dans la figure 15.
   Ces cellules dendritiques sont mises au contact de cellules mononuclées (PBLo). L'activité CTL est mesurée après restimulation par les peptides antigéniques correspondants.
   Sur l'axe des abscisses, est représenté le rapport cellules effectrices / cellules cibles.

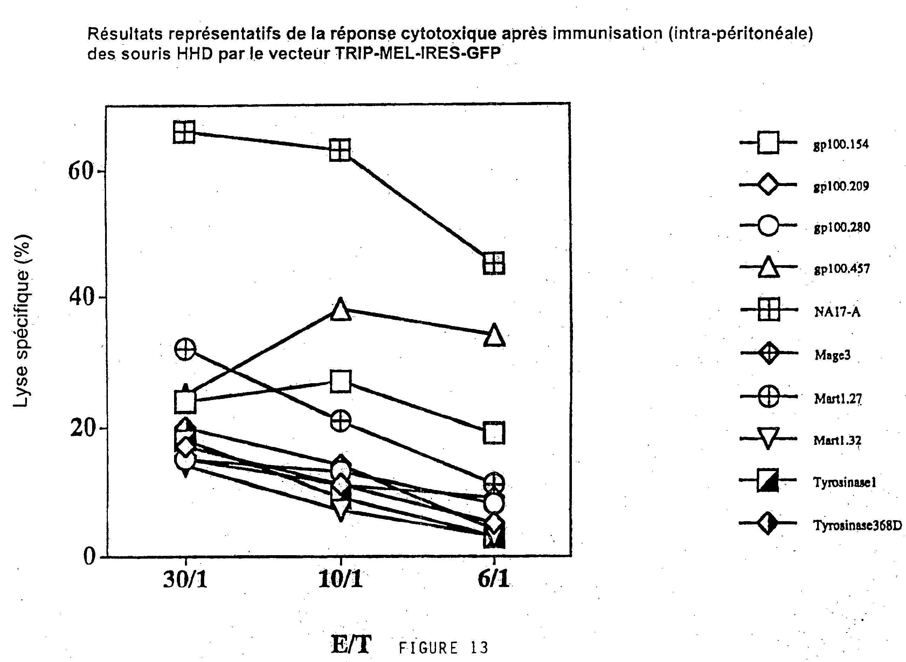

**FIG. 13** **:** Réponse cytotoxique après immunisation de souris avec le vecteur TRIP.MEL-IRES-GFP.
**FIG. 14** **:** Carte de restriction du vecteur pTRIP.MEL-IRES-GFP
   La souche E. coli contenant le vecteur pTRIP-MEL-IRES-GFP a été déposée le 20 Avril 1999 à la CNCM et a le numéro d'accession n° I-2185.
**FIGURE 15** **:** Séquences des épitopes CTLs HLA A2.1 spécifiques du mélanome inclus dans la construction polyépitopique du vecteur TRIP.MEL-IRES-GFP. Le soulignement des séquences du polyépitope a pour objet d'individualiser chaque épitope.

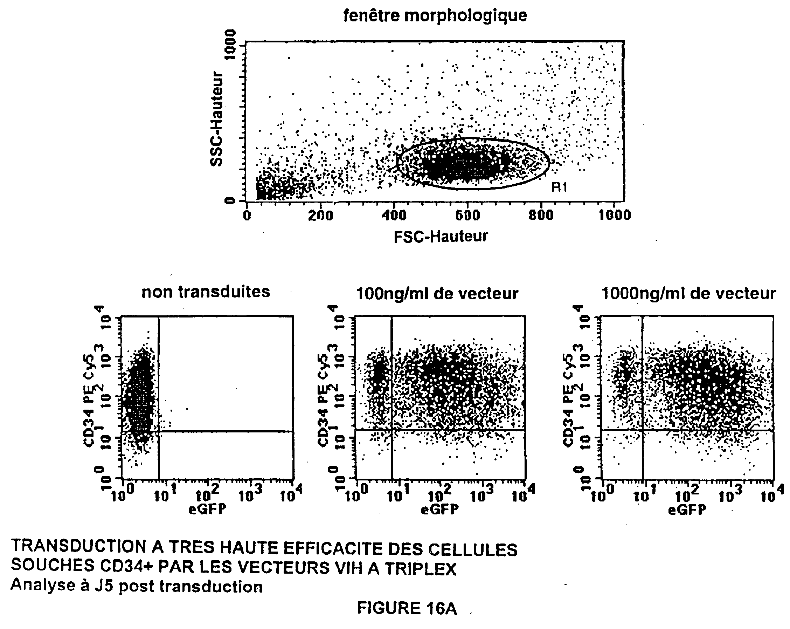

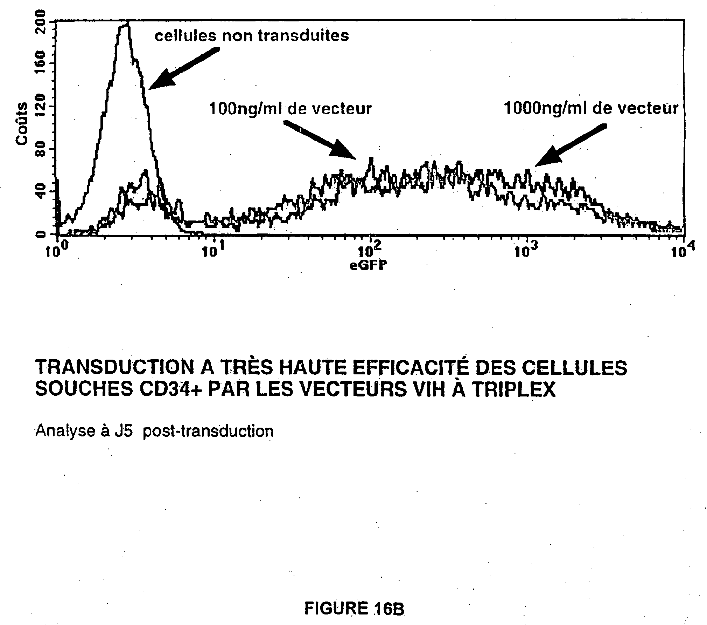

**FIGURE 16** **: Transduction à très haute efficacité** des **cellules souches CD34+ par les vecteurs VIH à triplex.**
   Analyse par cytométrie en flux FACS de la transduction du gène GFP dans les cellules souches hématopoïétiques CD34+ par le vecteur TRIP-GFP. Le pourcentage de cellules CD34+ transduites par le vecteur TRIP-GFP est supérieur à 85%. Cette efficacité est notablement plus élevée que les taux de transductions obtenus précedemment avec un vecteur VIH sans triplex (HR-GFP) dans les cellules CD34+ (Miyoshi H et al., Science 1999, 283. p 682-6).

### MATERIEL ET METHODES

### Construction des plasmides vecteurs:

Les plasmides pTRIP-LacZ et pTRIP-EGFP dérivent de la construction pHR'CMVIacZ (Naldini et al, 1996). Le gène rapporteur LacZ de pHR'CMVIacZ a été remplacé par l'ORF de la protéine autofluorescente EGFP. Le gène de l'EGFP a été amplifié par PCR à partir du plasmide pEGFP-N1 (Clontech) grâce à la polymérase thermostable fidèle Pfu (Stratagene). Les séquences des amorces PCR utilisées sont les suivantes:
Bam EGFP: 5' cc gga tcc cca ccg gtc gcc acc 3'
Xho EGFP: 5' cc ctc gag cta gag tcg cgg ccg 3'

L'amplification PCR a été réalisée en 30 cycles dans les conditions suivantes:
- Dénaturation 95 °C, 30 sec
- Hybridation 50 °C, 1 min
- Elongation 75°C, 30 sec

Les sites de restriction BamHI et XhoI ont été ajoutés en 5' et 3' respectivement du fragment PCR EGFP de manière à l'insérer de façon orientée dans le fragment vecteur pHR'CMV, lui même digéré par BamHI et XhoI. L'insertion du fragment PCR EGFP, par des technologies classiques d'ADN recombinant (Maniatis et al, 1983), a généré le plasmide pHR-EGFP.

Un fragment de 184 bp correspondant à la région centrale du génome VIH-1 et comprenant les boites cis-actives cPPT et CTS, responsables de la formation du triplex au cours de la rétrotranscription du VIH, a été inséré dans le site Clal des plasmides pHR-EGFP et pHR'CMVIacZ , en amont du promoteur CMV. La région centrale triplex a été amplifiée par PCR à partir de plasmides proviraux complets du génome HIV-1 LAI comprenant la séquence triplex sauvage (pBRU3; Chameau et al, 1991), mutée dans la séquence cis-active de terminaison CTS (pCTS; Chameau et al, 1994) ou encore mutée dans la séquence cis-active d'initiation centrale cPPT (p225; Chameau et al, 1992).

Les séquences des amorces PCR utilisées sont les suivantes:
Nar/Eco TRIP+: 5' gtc gtc ggc gcc gaa ttc aca aat ggc agt att cat cc 3'
Nar TRIP-: 5' gtc gtc ggc gcc cca aag tgg atc tct gct gtc c 3'

Les conditions de réaction PCR étaient identiques à celles décrites précédemment.

Les fragments PCR Triplex, digérés par Narl, ont été insérés au site ClaI des plasmides pHR GFP et pHR'CMV LacZ par ligation/digestion compétitive T4 DNA ligase/ClaI afin d'éliminer au cours de la ligation le vecteur recircularisé sur lui même. L'orientation de l'insertion est analysée par digestion XhoI/EcoRI, le site Eco RI ayant été introduit dans l'amorce PCR 5' Nar TRIP+.

Les plasmides résultants sont dénommés pTRIP.EGFP dans l'orientation correcte du triplex et pTRIPinv.EGFP dans l'orientation inverse, non fonctionnelle. Les vecteurs comprenant une version mutée du triplex sont dénommés pTRIP X .EGFP, X correspondant au code du virus mutant de départ (AG, D, CTS ou 225) (Chameau et al J. Mol. Biol. 1994, Charneau et al J. Virol 1992). A partir des différents plasmides pTRIP.EGFP ou pTRIP X .EGFP, le gène EGFP a été remplacé par Lac Z par échange orienté XhoI/BamHI. Les plasmides résultants sont dénommés respectivement pTRIP.Z, pTRIPinv.Z, pTRIP CTS.Z, pTRIP 225.Z.

### Constructions des vecteurs HR luc et TRIP luc

Le fragment BamHI-EGFP-Xhol des vecteurs RH GFP et TRIP GFP, a été remplacé par le fragment BamHI-Luc-XhoI du plasmide pGEM-luc (Promega) codant pour la luciférase.

### Production des particules vecteurs non infectieuses

La production des vecteurs HIV a été réalisée selon une modification du protocole décrit dans Naldini et al, 1996. Les particules vecteur ont été produites par co-transfection transitoire au phosphate de calcium de cellules humaines 293T (ATCC), cultivées en milieu DMEM (ICN), SVF 10%, pénicilline, streptomycine. Des boites demi-confluentes de 175 cm² ont été transfectées simultanément par trois plasmides:
- 15µg de plasmide codant pour l'enveloppe du virus de la stomatite vésiculaire (VSV), pMD.G (Naldini et al, 1996)
- 30µg de plasmide d'encapsidation, pCMVΔR8.2 (Naldini et al, 1996) ou pCMVΔR8.91 (Zufferey et al, 1997).
- et 30µg des différents plasmides vecteurs pHR ou pTRIP.

Les co-précipités phosphate-calcium/ADN ont été laissés au contact des cellules pendant 24 heures, le milieu a ensuite été collecté chaque 24 heures jusqu'au jour 3 post transfection. Les débris cellulaires des surnageants vecteurs ont été éliminés par centrifugation basse vitesse. Les surnageants vecteur ont été conservés par congélation à -80°C.

### Concentration des particules vecteur

L'utilisation de l'enveloppe VSV-G, très stable, pour pseudotyper les particules vecteur permet leur concentration par ultracentrifugation. Les surnageants vecteurs, collectés selon la méthode décrite ci-dessus, ont été ultracentrifugés dans des tubes à fond coniques (Beckman) de 30ml, 90 min à 17000 rpm à +4°C dans un rotor SW 28 (Beckman). Les culots ont été ensuite repris dans 190†µl de PBS, centrifugés 5 min à 2000rpm afin d'enlever les débris non resuspendables, aliquotés et congelés à -80°C.

### Transduction de cellules en culture

Les cellules Hela (ATCC) ont été transduites par addition de surnageants vecteur, ultracentrifugés ou non, en présence de 10 µg/ml de DEAE Dextran. Les cellules Hela sont cultivées en milieu DMEM (ICN) supplémenté par 10% de sérum de veau foetal (SVF). Les cellules HeLa ont été étalées à 20 000 cellules/puits en plaque 96 puits, la veille de l'infection puis transduites dans un volume de 200µl final. Les innoculum de vecteur ont été normalisés selon la concentration de protéine de capside (P24), calculée selon un test ELISA commercial (DuPont). L'efficacité de transfert de gène a été mesurée, selon les expériences 24 à 48 heures post-infection. Le taux de transduction des vecteurs exprimant le gène rapporteur LacZ était révélé soit par une coloration XGaI in situ (Chameau et al, 1992), soit par une réaction luminométrique selon un kit commercial (Boehringer) en suivant les instructions du fournisseur. Dans le cas de vecteurs exprimant le gène rapporteur EGFP, le taux de transduction a été évalué qualitativement par observation directe des cellules vivantes au microscope à fluorescence, sur le canal FITC. La quantification du nombre de cellules exprimant le marqueur EGFP a été réalisée par cytométrie de flux (canal FITC). Le dosage de la protéine EGFP a pu être réalisé par mesure de la fluorescence sur extrait cellulaire. Les plaques de cultures à 96 puits ont été rincées deux fois au PBS puis lysées par 100µl de PBS 1% NP40. La fluorescence EGFP a été lue grâce à l'utilisation d'un fluorimètre en plaque (modèle Victor, Wallac) avec un filtre d'exitation à 475 nm et un filtre d'émission à 510 nm.

Des cellules Hela arrêtées dans leur cycle cellulaire en transition G1/S ont été préparées selon les mêmes conditions que précédemment avec un traitement préalable de 24 heures avant la transduction par 4 µM d'aphidicoline (Sigma). Dans ces conditions, plus de 95% de l'incorporation de thymidine tritiée était inhibée.

### Transduction ex vivo de cellules primaires

Des cultures primaires de cellules de moelle épinière de rat ont été préparées de la façon suivante: des moelles d'embryons de rat de 13 à 14 jours ont été disséquées sous la loupe binoculaire. Les tissus ont été maintenus en milieu L15 (Gibco) supplémenté par 3.6 mg/ml de glucose pendant toutes les étapes. Les cellules nerveuses ont été dissociées par incubation dans la trypsine (0.05% w/v) pendant 15 min à 37°C. La digestion trysique a été inhibée par l'addition de 10% de sérum de veau foetal (SVF) et centrifugation à basse vitesse. Le culot cellulaire a été repris dans du milieu L15, 3.6 mg/ml de glucose contenant 100 µg/ml de DNasel (Boehringer), par agitation mécanique douce. Les cellules ont été collectées par centrifugation basse vitesse à travers un coussin de BSA à 4% (w/v).

Les cellules spinales ont été ensemmencées dans des plaques à 24 puits contenant des lamelles de verre de 12 mn de diamètre et recouvertes de poly-DL-Ornithine (6 µg/ml) et de laminine (3 µg/ml). Les cultures cellulaires ont été entretenues dans du milieu neurobasal (Gibco) contenant du supplément B27, 2% de SVF, 0.5 mM L-Glutamine, 25 µM de beta-mercaptoethanol et 25 µM de L-Glutamate. Les cultures ont été traitées après 24 heures par 10 µg/ml de 5' fluorodeoxyuridine pour prévenir la colonisation de la culture par des cellules non neuronales.

### Transduction in vivo de l'EGFP dans le cerveau de rat

Les vecteurs exprimant la protéine marqueur EGFP ont été utilisés pour les expériences *in vivo.*

Des injections dans le cerveau de rat avec 2µl de vecteur HR.EGFP ou TRIP.EGFP, ont été réalisées sur des rats OFA spague dawley de 5 semaines. Dans un premier temps, les rats ont été endormis par injection intrapéritonéale d'Imagene 500 (Rhône Merieux). Les injections ont été réalisées dans le striatum de chaque hémisphère à l'aide d'un guide stéréotaxique, avec une aiguille Hamilton de 5 µl, à la vitesse de 2µl / 5min. Les rats ont été sacrifiés une semaine ou plus après injection, par perfusion au PBS puis au paraformaldehyde 2% (PFA). Les cerveaux ont ensuite été prélevés et découpés pour ne garder que la partie contenant le point d'injection, visible par la lésion laissée par l'aiguille. Une post fixation au PFA 2% a été réalisée toute la nuit, suivie d'une cryoprotection au sucrose 20% puis 30%. Les cerveaux ont été ensuite recouverts de tissus-tek, congelés dans la carboglace et stockés à -80°C. Des coupes sériées de 14 µm ont été réalisées à l'aide d'un cryostat puis observées avec un microscope confocal.

### Comparaison in vitro et in vivo des vecteurs HR luc et TRIP luc

Les cellules HeLa ont été étalées, un jour avant la transduction, à 20000 cellules par puits, en plaques 96 puits. Les transductions ont été réalisées avec la même quantité de particules vecteurs, normalisée sur le contenu en p24 des préparations: 1 ng de p24 par puits, en triplicate, en présence de 10µg/ml de DEAE-dextran. Deux jours après transduction, l'activité luciférase a été mesurée à l'aide d'un kit Promega (suivant les instructions du fabriquant) et d'un appareil de mesure en microplaque Wallac (Victor).

Des injections vecteurs dans le striatum de cerveau de rat OFA spague dawley et de souris C57B6 ont été réalisées. 2 µl d'une préparation de HR luc ou TRIP luc, contenant 25 ng de p24 ont été injectés (n=4). Les animaux ont été sacrifiés 4 jours après, les striatum ont été prélevés et l'activité luciférase mesurée par la même technique que précédemment, en mesurant en parallèle la quantité de protéines totale (kit Pierce).

### EXEMPLES

### 1 Aspects fondamentaux. Import nucléaire du complexe de pré-intégration

### HIV-1: rôle du triplex central

Le mécanisme de rétrotranscription du virus HIV diffère de celui des rétrovirus oncogènes par la synthèse du brin-plus (brin +) en deux moitiés distinctes (figure 1). Un segment aval est initié à une copie centrale du polypurine tract (cPPT), caractéristique des génomes lentiviraux. La synthèse du brin-plus amont se termine après un déplacement de brin discret au centre du génome. Le blocage du déplacement de brin par la reverse transcriptase est gouverné par une nouvelle séquence cis-active du génome HIV: le CTS (Central Termination Sequence). Le produit final de la rétrotranscription dés lentivirus est donc un ADN linéaire portant une structure centrale en chevauchement de brin (triplex central) sur une centaine de nucléotides (Chameau et al, 1994). La mutagenèse dirigée du cPPT ou du CTS peut abolir l'initiation ou la terminaison centrale de la synthèse du brin-plus. Dans les deux cas, les virus mutants, dont l'ADN est dépourvu du triplex central, sont défectifs pour la réplication.

L'analyse du défaut réplicatif des mutants d'initiation et de terminaison centrale a montré que le cycle réplicatif des mutants d'initiation ou de terminaison centrale de la rétrotranscription avorte lors d'une étape postérieure à la synthèse de l'ADN viral et postérieure au routage du complexe de rétrotranscription vers l'enveloppe nucléaire. Lorsqu'on analyse la structure de l'ADN viral présent dans les cellules infectées, on s'aperçoit que les phénotypes des mutants d'initiation et de terminaison sont semblables. Dans les deux cas, en effet, la quantité globale d'ADN rétrotranscrit n'est pas affectée par des mutations dans le cPPT ou le CTS. On observe en revanche une accumulation d'ADN linéaire non intégré, avec, dans le même temps, très peu de provirus intégrés ou de cercles à 1 ou 2 LTRs formés. Des expériences de fractionnement noyaux/cytoplasme et de perméabilisation du noyau ont ensuite montré que ces molécules d'ADN linéaire sont associées au noyau, mais que leur intégration et/ou leur circularisation ne peuvent se produire qu'après dissolution de l'enveloppe nucléaire, ce qui indique bien que l'ADN viral des mutants est retenu à l'extérieur de cette enveloppe. De plus des expériences d'attaque nucléasique de noyaux purifiés de cellules infectées par la DNasel immobilisée sur billes d'or démontre encore l'accumulation des ADNs linéaires mutants à la face cytoplasmique de la membrane nucléaire. Enfin, une quantification précise de la capacité intégrative des molécules d'ADN linéaires pourvues ou non d'un triplex central natif a récemment montré que le triplex central n'influence pas l'intégration de l'ADN linéaire dans une cible ADN hétérologue in vitro.

Le défaut réplicatif des virus mutants pour l'initiation ou la terminaison centrale de la rétrotranscription concerne donc l'import nucléaire de leur complexe de pré-intégration, et plus précisément l'étape de translocation à travers les pores nucléaires. Les lentivirus, et le virus HIV en particulier, ont développé une stratégie originale de rétrotranscription dont le but est de créer le triplex au centre des molécules d'ADN non intégrées, déterminant indispensable à l'entrée du génome viral dans le noyau d'une cellule en interphase. Ce mécanisme distingue les lentivirus de tous les autres rétrovirus dont l'accès de l'ADN au site d'intégration dépend de la désorganisation de la membrane nucléaire pendant la mitose.

### 2. Génération de vecteurs lentiviraux contenant les séquences cis-actives responsables de la formation du triplex

### 2-1 Principe et intérêt des vecteurs lentiviraux

La génération de vecteurs lentiviraux efficaces suppose la connaissance des déterminants responsables de l'import nucléaire actif et donc de l'infection des cellules non mitotiques.

La découverte de l'implication du triplex dans l'import nucléaire du génome HIV-1 a des conséquences importantes pour la construction de vecteurs lentiviraux efficaces. Elle suppose la conservation, au sein de la construction vecteur, des séquences actives en cis et responsables de la formation du triplex ADN lors de la rétrotranscription lentivirale. L'application en vectorologie de ce travail fondamental consiste à adjoindre dans les constructions de vecteurs lentiviraux la région centrale cPPT-CTS de façon à créer la structure ADN triplex lors de la rétrotranscription du génome vecteur. Il est à noter que de nombreuses tentatives de construction de vecteur lentiviraux, basées sur le même principe que les vecteurs dérivés des oncovirus (en général du MoMLV), se sont révélées très décevantes, au moins en terme de titre infectieux. Ces vecteurs sont des vecteurs de remplacement, c'est à dire que l'ensemble du génome rétroviral est délété puis remplacé, entre les deux LTRs et la séquence d'encapsidation, par le gène reporteur ou le gène d'intérêt thérapeutique (Miller et al, 89). Selon les inventeurs, ce type de construction n'est pas optimum dans le cas de vecteurs lentiviraux du fait de la nécessité de la région centrale cPPT-CTS pour l'import nucléaire de l'ADN viral. Toutefois, des vecteurs HIV construits sur le même principe que les vecteurs rétroviraux dérivés d'oncovirus, mais pseudotypés par l'enveloppe très fusiogène du virus de la stomatite vésiculaire (VSV-G) et concentrés par ultracentrifugation, permettent la transduction *in vivo* de neurones de rat (Naldini et al, 1997; Blomer et al, 1997), du foie et du muscle différencié (Kafri et al, 1997). Pourtant, des expériences de complémentation de xénogreffes d'épithelium pulmonaire humain par ces vecteurs HIV codant pour le gène CFTR (mucoviscidose) se sont révélées très décevantes. L'essentiel de l'ADN vecteur dans ce tissu resterait sous forme d'ADN linéaire non intégré, révélant ainsi un défaut probable d'import nucléaire (Goldman et al, 1997; voir le chapitre "influence du triplex central sur le taux d'import nucléaire de l'ADN vecteur").

### 2.2. Construction et production des vecteurs HIV "à triplex"

Afin de tester l'importance de la structure triplex dans un système vecteur, les inventeurs ont pris pour base les constructions décrites par Naldini et al. Dans ce système (figure 2), les particules vecteur HIV sont produites par co-transfection transitoire de trois plasmides: un plasmide d'encapsidation exprimant l'ensemble des protéines virales à l'exception de l'enveloppe du HIV. un plasmide exprimant l'enveloppe VSV-G et un plasmide vecteur, pHR-CMV LacZ, comprenant les LTRs du HIV, le signal bipartite d'encapsidation du VIH et une cassette d'expression de LacZ. Dans un premier temps, le gène rapporteur LacZ a été remplacé par un gène codant pur une version très fluorescente de la EGFP (E Green Fluorescent Protein), plus pratique pour les études de transduction in vivo. La région centrale du génome HIV-1 LAI comprenant les séquences cis-actives cPPT et CTS, responsables de la formation du triplex, a été amplifiée par PCR puis insérée au site ClaI, dans la construction vecteur pHR-EGFP. L'insertion d'un triplex sauvage dans l'orientation correcte génère le vecteur pTRIP-EGFP; dans l'orientation inverse (non fonctionnelle), le vecteur pTRIPinv-EGFP.

### 2.3. Test rapide et sensible de détection de virus helper dans les préparations de vecteurs lentiviraux :

### Absence de virus infectieux helper dans les "surnageants vecteur"

La production de particules vecteur à partir de trois plasmides indépendants et possédant un minimum de séquences homologues permet de minimiser la probabilité de générer un virus helper compétent pour la réplication. De plus, la construction d'encapsidation a été délétée pour l'enveloppe du VIH et pour l'ensemble des gènes dits accessoires à la réplication (Vif, Vpr, Vpu, Nef). Le génome vecteur encapsidé ne contenait plus du VIH que les 2 LTRs, les séquences nécessaires à l'encapsidation et la séquence triplex. Toutefois, chaque stock de vecteur a été testé pour la présence de virus helper infectieux. Des cellules MT4 ont été infectées en triplicate, sur microplaque, pendant une nuit, puis lavées extensivement et remises en culture pendant 5 jours afin d'amplifier l'innoculum. Des cellules indicatrices P4 (HeLa CD4 LTR-LacZ) ont ensuite été infectées avec les cellules MT4 et leur surnageant pendant 3 jours afin de détecter les particules infectieuses produites. Enfin, une coloration X-gal in situ a été réalisée. De cette façon, toute particule infectieuse produite est détectable sous forme d'un syncitia bleu. Ce protocole sensible permet de détecter un innoculum de VIH de 0,25 pg de P24, soit environ 3200 particules physiques. Sachant que dans le cas de HIV, on estime qu'une seule particule sur 1000, ou même sur 10 000, est infectieuse, le protocole devait probablement permettre de détecter une particule infectieuse unique.

Les surnageants vecteur sont systématiquement dépourvus de particules infectieuses HIV.

### 2.4. Effet du triplex sur l'efficacité de transduction par les vecteurs in vitro

Dans un premier temps (figure 3), l'effet de l'insertion du triplex central sur la transduction de cellules HeLa a été mesuré. Des cellules HeLa ont été infectées avec un surnageant de transfection de vecteur le triplex central sauvage (TRIP GFP), un vecteur sans cette séquence (HR GFP), ou avec une séquence triplex mutante (TRIP GFP D). Le mutant D est un mutant du cPPT qui empêche l'initiation centrale du brin + et donc la formation du triplex central (figure 3). Les infections ont été réalisées avec des surnageants contenant la même quantité de particules normalisées sur la quantité de protéine de capside p24.

La transduction dé la GFP dans les cellules Hela a été augmentée en présence d'un triplex sauvage et était ramenée au taux basal en présence d'un triplex non fonctionnel.

Ce gain de titre peut être quantifié grâce à l'utilisation du gène rapporteur LacZ (figure 4). Ces cellules ont été transduites en triplicate en normalisant par référence à la quantité de protéine p24. Les transductions ont été réalisées sur des cellules bloquées ou non en division à l'aphidicoline, qui bloque les cellules en G1/S. Les vecteurs utilisés étaient, HRZ (sans triplex), TRIP Z (avec triplex), TRIP Z inv (la séquence triplex est orientée en sens inverse, elle est non fonctionnelle et ne conduit pas à la formation d'un triplex central).

Figure 4A: Le gain de transduction de la ßgal par des vecteurs contenant le triplex est de 6 à 10 fois. Il est perdu lorsque le triplex n'est pas formé (TRIP Z inv).

Figure 4B: L'effet du triplex sur la transduction de ßgal est indépendant de la division cellulaire: des résultats similaires sont obtenus sur des cellules en division ou bloquées à l'aphidicoline.

Par ailleurs, les mêmes résultats sont obtenus sur cellules HeLa, que le plasmide d'encapsidation utilisé lors de la production des particules vecteur soit ou non délété dans les gènes accessoires Vif, Vpr, Vpu, Nef.

### 2.5. Effet du triplex sur l'efficacité de transduction par les vecteurs ex vivo

L'impact du triplex sur la transduction de la EGFP dans des cellules primaires non mitotiques a par la suite été mesuré. Des explants primaires de moelle épinière de rat enrichis en neurones ont été transduits avec des surnageants de vecteur ultracentrifugés. Les transductions ont été réalisées avec moins de 10µl de vecteur ultracentrifugé, contenant de la même nombre de particules, normalisé sur le nombre de ng de protéine de capside p24.

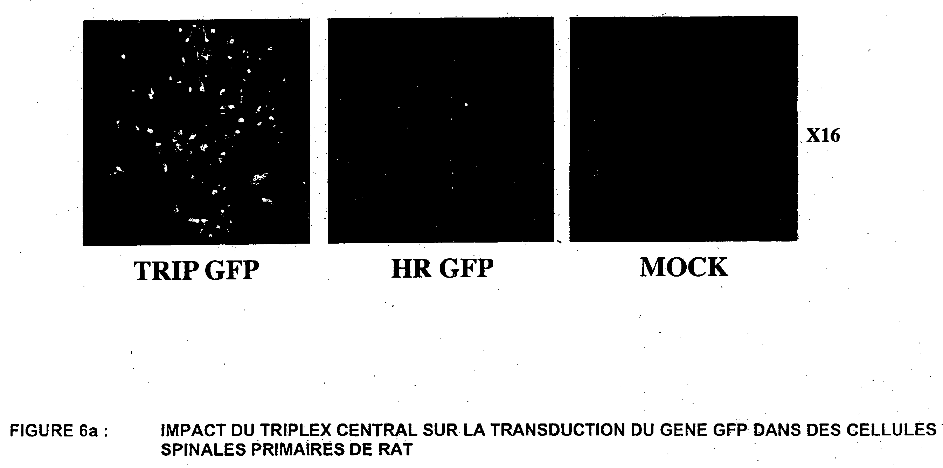

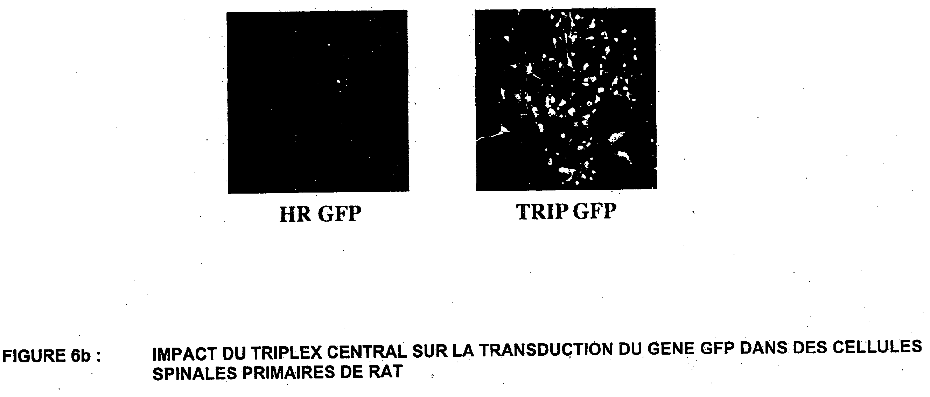

Figure 6: le vecteur possédant une séquence triplex transduit un beaucoup plus grand nombre de cellules d'explants primaires de moelle épinière de rat que le vecteur sans triplex.

### 2.6. Impact du triple sur la transductions in vivo dans le cerveau

L'effet du triplex sur la transduction de la EGFP *in vivo* a ensuite été mesuré par injection directe dans le cerveau de rat. Le même volume (2µl) de surnageant de vecteur avec ou sans triplex contenant la même quantité de protéine p24 a été injecté dans le striatum. Alors qu'un grand nombre de cellules transduites ont été détectées chez les rats injectés avec le vecteur avec triplex (figure 7a), il a été possible de détecter de peu de cellules exprimant la EGFP dans le cerveau des rats injectés avec le vecteur sans triplex, et ce même au point exact de l'injection, visible par la lésion laissée par l'aiguille.

Sur la figure 7b la construction de vecteurs HIV (avec ou sans séquence ADN triplex) qui expriment le gène reporter luciférase (HR Luc et TRIP.Luc) a permis de quantifier précisément l'impact sur la transduction de gènes dans le cerveau. In vitro une augmentation d'un facteur 8 est observée dans les cellules HeLa (Figure 7-b1). Un bénéfice analogue est obtenu après injection directe in vivo dans le striatum de cerveau de rat (Figure 7-b2) ou de souris (Figure 7-b3).

### 2.7. Impact du triplex sur /'import nucléaire du génome vecteur

Un test permettant de suivre au cours du temps l'ensemble des formes ADN vecteur dans la cellule transduite a été mis au point par les inventeurs : ADN linéaire, cercles à 1 ou 2 LTRs mais aussi provirus intégré. Ce test est basé sur la détection par Southern de l'ADN viral selon une stratégie de coupure et de choix de sonde permettant de différencier les différentes formes d'ADN rétroviral (voir figure 8). L'ADN total des cellules infectées ou des cellules transduites par les vecteurs est digéré par une ou deux enzymes de restriction de façon à dégager un fragment interne, commun à toutes les formes d'ADN rétroviral ou d'ADN vecteur présentes dans les cellules (ADN linéaire non intégré, ADN circulaire à un ou deux LTRs et provirus intégré). Dans le cas du vecteur, les enzymes choisies sont Eco NI et Ava II. En utilisant pour sonde un fragment généré par PCR chevauchant exactement le site Eco NI, plusieurs bandes correspondant aux différentes formes d'ADN apparaissent. Le fragment interne permettra de calculer après quantification au Phosphorimager l'ADN total vecteur présent dans les cellules. Un fragment de 1.16 Kb correspond au fragment distal de l'ADN linéaire non intégré, un autre de 3.3 Kb correspond aux cercles non intégrés. Après quantification des signaux avec un phosphorimager, le taux d'import nucléaire est indiqué par le pourcentage d'ADN viral intégré et sous forme circulaire (ADNs viraux nucléaires) par rapport à l'ADN linéaire cytoplasmique. Les premiers blots préliminaires ont montré un profil d'ADN intracellulaire caractéristique d'un défaut d'import nucléaire dans le cas des vecteurs dépourvus de triplex ou dont la région centrale du génome HIV-1 a été insérée à l'envers. En effet, l'intensité du signal correspondant à l'ADN linaire était équivalent à celle du signal ADN total, 48 heures après infection. En d'autres termes, le processing de l'ADN vecteur est majoritairement bloqué au stade linéaire non intégré, très peu de molécules s'intègrent (figure 9). A l'inverse, dans le cas des vecteurs possédant un triplex, il ne subsiste que peu d'ADN linéaire à 48 heures, indiquant que leur majeure partie a été importée dans le noyau de la cellule transduite, puis s'est intégrée.

### 2.8. Etude de l'effet de position du trilex ADN dans la construction vecteur

On trouve chez tous les lentivirus les séquences cis-actives cPPT et CTS, responsables de la formation du triplex lors de la rétrotranscription. Dans tous les cas, ce triplex se trouve à quelques nucléotides près au centre du génome ADN linéaire. Cette position centrale du triplex pourrait être importante pour le fonctionnement optimal de ce déterminant de la translocation à travers le pore nucléaire. Les constructions vecteur réalisées, la séquence triplex a été insérée juste en amont de l'unité transcriptionnelle du gène rapporteur. Selon la taille du gène rapporteur, ce triplex se trouvait plus ou moins près du centre du génome ADN linéaire vecteur. Dans le cas du gène rapporteur EGFP (0.7 Kb), le triplex est très proche du centre de la construction; alors que dans le cas de LacZ (3.1 Kb), il en est plus éloigné (figure 2). Dans les deux cas, la présence du triplex a induit un gain en titre important des surnageants vecteurs. Il existe donc une certaine "flexibilité" dans la position du triplex sur le génome vecteur. Toutefois, les vecteurs codant pour la EGFP se sont avérés clairement plus efficaces que ceux codant pour LacZ. Il est donc possible qu'un triplex placé idéalement permette un gain en titre supplémentaire. Afin de tester cette hypothèse, les inventeurs ont entrepris de cloner, à la place des gènes rapporteurs, une banque de fragments de taille aléatoire (digestion partielle Sau3A), la distribution en taille des fragments clonés étant analysée avant et après transduction de cellules cibles. Dans le cas où la position centrale du triplex serait importante pour sa fonction, la contrainte de construction d'un vecteur symétrique par rapport au triplex serait importante. Il est possible de contourner cet obstacle en insérant l'unité transcriptionnelle du vecteur dans la région U3. Après rétrotranscription, le transgène sera dupliqué de part et d'autre du triplex avant de s'intégrer, respectant ainsi une position exactement centrale du triplex.

### 2.9. Transfert in vivo dans différents tissus différenciés

La capacité des vecteurs lentiviraux "triplex" à transduire efficacement et stablement les tissus différenciés affectés dans diverses pathologies génétiques est étudiée. Le potentiel de ces vecteurs dans le cerveau, et dans différents tissus tels que le muscle, l'épithélium pulmonaire et le foie chez le rat ou la souris. Des réponses qualitatives peuvent être relativement rapidement obtenues grâce à l'utilisation du gène rapporteur EGFP. Des mesures quantitatives de l'impact du triplex sur le taux de transduction de ces tissus sont permises en utilisant le gène rapporteur luciférase. Par ailleurs, la capacité de ces vecteurs à transduire les cellules souches totipotentes du tissu hématopoïétique humain peut être évaluée, soit à partir de cellules CD34+ purifiées, soit à partir de cellules de sang de cordon total.

### 2.10. Transfert de gènes à haute efficacité dans des cellules souches hématopoïétiques par les vecteurs HIV à triplex.

Les cellules souches hématopoïétiques sont des cibles d'importance majeure pour le traitement d'un grand nombre de désordres génétiques liés au sang, musculaires ou neurologiques et de maladies infectieuses. La difficulté majeure pour le transfert de gène par vecteurs rétroviraux dérivés d'oncovirus comme le MoMLV dans ces cellules est qu'elles se divisent très peu et que l'induction de mitoses par un traitement cytokine s'accompagne en général d'une perte de leur totipotence. Sur la figure 16, les résultats de la transduction du gène GFP dans les cellules souches CD34 par le vecteur TRIP-GFP montrent une expression de la GFP dans plus de 85% des cellules. L'efficacité de transduction des cellules souches CD34 par le vecteur sans triplex HR-GFP est très inférieure (Miyishi H et al., Science 1999, 283, p 682-6). Les cellules souches CD34 étant transduite dès leur purification, leur capacité clonogénique reste intacte.

### 2.11. Utilisation des vecteurs lentiviraux à séquence triplex pour la transduction de cellules embryonnaires : Application à la construction d'animaux transgéniques ou de lignées cellulaires modifiées.

Les vecteurs rétroviraux sont potentiellement des outils intéressants pour la construction d'animaux transgéniques via la transduction des oeufs (Rubenstein et al., 1986, PNAS, 83, p 366-368) ou des cellules ES (Friedrich and Soriano, 1991, Genes Dev. 5, p 1513-1523). L'utilisation des vecteurs lentiviraux est susceptible d'augmenter l'efficacité de transduction de ces cellules totipotentes. Nos résultats préliminaires de transduction de cellules embryonnaires de souris par le vecteur TRIP-GFP montrent une grande efficacité de transfert du gène GFP mais aussi une extinction complète de la transcription du transgène GFP. Certaines séquences virales en particulier le primar binding site (PBS) sont suspectées d'intervenir dans cette extinction d'expression. Afin de contourner cet obstacle, des vecteurs autodélétants pour ces séquences virales, vasés sur le système de recombinaison spécifique CRE/Lox (Choulika et al., 1996, J. VIROL, 70, p 1792-98), ont été construits.

### 2.12. Composition immunogène à applications prophylactiques et/ou thérapeutiques :

### Une nouvelle stratégie d'immunisation: vecteurs lentiviraux à triplex.

Le rôle des lymphocytes T cytotoxiques dans la réponse antitumorale et antivirale a été documenté, dans de nombreux systèmes expérimentaux murins mais aussi chez l'homme. Differentes stratégies vaccinales visent à induire une réponse cytotoxique protectrice contre les tumeurs ou agents infectieux. Les lentivirus ont la capacité de traverser les pores nucléaires et sont, en conséquence, de bien meilleurs vecteurs de transduction de cellules. La transduction des cellules in vitro et/ou in vivo peut conduire à la présentation par ces cellules, des épitopes d'antigènes tumoraux et/ou viraux qui par la suite induiront une immunité cellulaire spécifique. Pour ces raisons les inventeurs ont étudié la capacité immunogénique des vecteurs lentiviraux à triplex recombinants en utilisant soit les cellules dendritiques transduites in vitro soit l'administration directe in vivo chez des souris "humanisées" par l'expression de HLA-A2.1 (Pascolo S. et coll. 1997). Cette souris HHD "HLA-A2.1 pure" est le meilleur modèle animal pour l'étude de la réponse cytotoxique HLA-A2.1 restreinte et a été proposée pour réaliser des études précliniques d'immunothérapie. L'ensemble de nos résultats montre clairement la capacité immunogénique des vecteurs lentiviraux à triplex contenant les épitopes tumoraux et de ce fait les vecteurs lentiviraux à triplex représentent une nouvelle stratégie d'immunothérapie.

### Etude initiale : comparaison de différentes stratégies vaccinales

En utilisant les souris HHD, différentes stratégies vaccinales ont été préalablément comparées. Sélectionnant arbitrairement 5 épitopes tumoraux les inventeurs ont comparé cinq stratégies d'immunisation applicables en clinique humaine: (i) peptides synthétiques en adjuvant incomplet de Freund, (ii) lipopeptides, (iii) particules recombinantes Ty de levure dans lesquelles, de manière indépendante les épitopes ont été fusionnés en C-terminal à la protéine P1. (iv) administration intramusculaire d'ADN-nu codant la glycoprotéine du virus de l'hépatite B fusionnée aux épitopes dans sa portion pré-S2, (v) injection intra veineuse de cellules dendritiques chargées de peptides après expansion et différenciation in vitro à partir des cellules de la moelle. Ayant observé que les injections de structures particulaires (Ty recombinantes de levure) ou d'ADN nu recombinant codant une glycoprotéine S du virus de l'hépatite B (Référence WO 95/11307 publié le 25/04/95) étaient les stratégies les plus efficaces d'induction de réponses cytolytiques, les inventeurs ont documenté, par insertion dans cette glycoprotéine d'un motif polyépitopique dérivé du mélanome (10 épitopes distincts), la possibilité d'induire simultanément chez toutes les souris testées des réponses cytolytiques contre 5 peptides épitopiques sur 10.

Les antigènes particulaires Ty ou d'ADN nu se sont avérés d'efficaces stratégies d'induction des réponses cytotoxiques. Cependant, la production sur une large échelle de particules Ty est difficile. De plus, il est à craindre que l'introduction d'épitopes hydrophobes et multiples dans le segment pré-S2 de la glycoprotéine du virus de l'hépatite B n'entraîne une forte réduction de la production de particules par les cellules CHO (mode de préparation de l'actuel vaccin contre l'hépatite). Les lentivirus (VIH-I) recombinants produits sous la forme de pseudotypes largement délétés, mais ayant conservé la séquence ADN triplex, ont la capacité de traverser la membrane nucléaire de cellules ne se divisant pas et représentent une stratégie vaccinale nouvelle et potentiellement plus efficace par rapport aux stratégies vaccinales citées ci-dessus.

### Matériels, méthodes et résultats

### Souris transgéniques

Les souris HHD, expriment une construction monocaténaire dans laquelle les domaines de présentation peptidique (a1, a2) de la molécule HLA-A2.1 sont covalemment associés en N-terminal, à la ß2-microglobuline humaine. Le domaine a3 et la partie intracytoplasmique de la molécule HLA-A2.1 sont remplacés par leur équivalent de la molécule H-2D^{b} (Pascolo S. et coll. 1997). Ces souris permettent d'étudier l'immunogénicité de peptides épitopiques et de différentes stratégies vaccinales d'une façon comparative.

### Construction du vecteur TRIP-MEL IRES GFP

Un vecteur bi-cistronique TRIP-IRES-GFP a tout d'abord été construit. Le site EcoRI du vecteur TRIP-IRES-GFP a été rempli avec l'ADN polymerase T4 créant le vecteur TRIP-DeltaE-GFP. Puis un fragment d'environ 1,2 kb BamHI- BstXI-SnaBI-EcoRI-IRES-EGFP-Xhol a été cloné à la place du fragment BamHI- EGFP-Xhol. Le fragment contenant l'IRES-EGFP (Internal Ribosome Entry Site) est un don gracieux du Dr Yongwon Choi (Rockfeller University, N.Y., USA). Un fragment contenant une séquence consensus Kozac et un polyépitope CTL mélanome a été généré par PCR, sur la matrice pBS mel poly avec la pfu polymerase et les oligonucléotides: 5BgIMlu Mel: 5' cc agatct acgcgt gcc acc atg gct gct ggt 3' 3RIMel: 5' CG GAATTC GAC CTA AAC GCA ACG GAT G 3'. Le fragment de PCR mel a ensuite été digéré par BgIII et EcoRI et cloné aux sites BamHI et EcoRI du vecteur TRIP-DeltaE-IRES-GFP, créant le vecteur TRIP-MEL-IRES-GFP.

### Efficacité de transduction in vitro des cellules dendritiques (CD) par les vecteurs lentiviraux GFP avec ou sans triplex

Les CD murines ont été obtenues à partir de la moelle de souris transgéniques HHD en presence d'IL4 et GM-CSF. Les CD humaines ont été obtenues à partir donneurs sains d'haplotype HLA-A2.1 (voir ci-dessous). Ces cellules ont été transduites par les vecteurs LV avec ou sans triplex en utilisant des concentrations differentes (75, 150, et 300ng-p24 de vecteur lentiviraux pour 5.10⁵ cellules).

L'expression de GFP dans les CD a été mesurée par FACS les jours 2, 5 et 10. Les valeurs en terme d'intensité moyenne de fluorescence correspondant à l'efficacité de transduction des cellules ont montré que les vecteurs lentiviraux à triplex ont une capacité de transduction 5 à 7 fois plus élévée des CD humaines par rapport aux vecteurs lentiviraux sans triplex.

### Induction des réponses CTL primaires en utilisant des cellules dendritiques humaines transduites par le vecteur TRIP-MEL-IRES-GFP

Les CD humaines immatures ont été obtenues à partir des donneurs sains d'haplotype HLA-A2.1 en présence de GM-CSF et IL13 (IDM, Paris, France). L'immunophénotypage de ces cellules par les anticorps monoclonaux contre CD1, CD4, HLA-ABC, HLA-DR, CD80, et CD86 a montré leur caractère immature avec une pureté en CD supérieure à 91%.

Les CD ainsi obtenues ont été transduites par le vecteur TRIP-MEL-IRES-GFP à la concentration de 100ng p24/vecteur pour 1.10⁶ cellules. L'efficacité de transduction des CD par TRIP-MEL-IRES-GFP a été étudiée en mesurant l'expression de GFP par FACS. Les cellules mononuclées (CMN) provenant de même donneur ont été stimulées par les CD préalablément transduites. Après trois stimulations, l'activité cytotoxique de ces cellules a été testée sur des cellules T2 chargées individuellement par 4 peptides épitopiques en utilisant un test CTL classique de 4 heures. Les peptides épitopiques Mage-3, gp100.154, GnTV/NA17/A, et Tyrosinase368-D ont été sélectionnés du fait de leur immunogénicité élévée dans les expériences antérieures.

Des réponses cytotoxiques spécifiques ont été observées contre tous les épitopes téstés. Le pourcentage de lyse observé pour chaque épitope est représenté dans la figure 12.

### Immunisation directe des souris HHD par le vecteur TRIP-MEL-IRES-GFP

Les souris HHD ont été immunisées par 2,5 µ/p24 du vecteur TRIP-MEL-IRES-GFP par souris en sous-cutané (SC), intra-veineux(IV) et en intra-péritonéal (IP). Le jour 11 d'immunisation, les cellules spléniques de chaque souris ont été stimulées individuellement par les peptides épitopiques de mélanome pendant 6 jours dont 2 jours en présence de 10% TCGF. L'activité lytique de ces cellules a ensuite été téstée sur des cellules RMAS chargées par les peptides correspondants ou sur des cellules HeLa-HHD transduites par le vecteur TRIP-MEL-IRES-GFP.

Les résultats obtenus pour chacune des souris sont répresentés en terme de lyse spécifique sur des cellules RMAS (Tableau 1) et sur des cellules Hela-HHD transduites (Tableau 2). Les meilleurs résultats ont été obtenus après administration du vecteur par voies SC et IP à la fois en termes de lyse et de nombre de reponses simultanément induites chez une souris donnée. Le fait remarquable est que la majorité des souris immunisées par voie IP développent des réponses cytolytiques contre tous les peptides épitopiques (figure 13).

**Tableau 1 : Réponse cytotoxique spécifique après immunisation par le vecteur TRIP-MEL-IRES GFP Cellules cibles : RMAS chargées par les peptides correspondants. Rapport Effecteurs/cible = 30**

| Lyses spécifiques obtenues après immunisation de souris HHD par LV contenant un polyépitope de mélanome Stimulation in vitro pour chaque souris individuelle SC au jour 8 en présence de TCGF et de peptide | | | | | | | | | | | | |
|---|---|---|---|---|---|---|---|---|---|---|---|---|
| | | | | | | | | | | | | |

| | **Souris** | **gp154** | **gp209** | **gp280** | **gp457** | **G-nTV** | **Mage-3** | **Mart1.27** | **Mart1.32** | **Tyro-1** | **Tyro368-D** | |
|---|---|---|---|---|---|---|---|---|---|---|---|---|
| **S.C.** | **1** | **25** | **4** | **13** | **8** | **54** | **17** | **17** | **4** | **4** | **10** | |
| | **2** | **44** | **1** | **5** | **11** | **89** | **10** | **11** | **4** | **3** | **6** | |
| | **3** | **39** | **0** | **19** | **21** | **81** | **29** | **26** | **6** | **4** | **0** | |
| **I.V.** | | | | | | | | | | | | |
| | **1** | **7** | **7** | **1** | **8** | **25** | **5** | **8** | **3** | **4** | **3** | |
| | **2** | **10** | **8** | **0** | **13** | **70** | **13** | **16** | **5** | **9** | **14** | |
| | **3** | **24** | **6** | **5** | **5** | **65** | **15** | **16** | **3** | **11** | **10** | |
| | **4** | **5** | **3** | **13** | **12** | **14** | **10** | **5** | **0** | **3** | **0** | |
| **I.P.** | | | | | | | | | | | | |
| | **1** | **30** | **10** | **2** | **3** | **57** | **9** | **6** | **4** | **3** | **2** | |
| | **2** | **63** | **8** | **7** | **17** | **72** | **11** | **19** | **9** | **7** | **7** | |
| | **3** | **21** | **7** | **8** | **16** | **72** | **14** | **32** | **0** | **7** | **7** | |

**Tableau 2 : Réponse cytotoxique spécifique après immunisation par le vecteur TRIP-MEL-IRES GFP Cellules cibles : HeLa-HHD transduites par le vecteur TRIP-MEL-IRES-GFP. Rapport Effecteurs/cible = 30**

| **Reponses cytolytiques des souris HHD immunisées en S.C, I.V. ou LP. avec le vecteur TRIP-MEL-IRES-GFP. Les résultats obtenus sur des cellules HeLa-HHD exprimant le polyépitope de mélanome.** | | | | | | | | | | |
|---|---|---|---|---|---|---|---|---|---|---|
| | **gp154** | **gp209** | **gp280** | **gp457** | **G-nTV** | **Mage-3** | **Mart1.27** | **Mart1.32** | **Tyro-1** | **Tyro368** |
| **S.C.** | **2** | **18** | **15** | **24** | **62** | **15** | **20** | **12** | **20** | **18** |
| **I.V.** | **8** | **10** | **15** | **23** | **50** | **14** | **29** | **10** | **10** | **18** |
| **I.P.** | **24** | **18** | **15** | **25** | **62** | **15** | **32** | **14** | **18** | **20** |

### Conclusion

Les résultats démontrent la capacité des vecteurs lentiviraux à (triplex d'induire des réponses immunitaires très efficaces. Leur pouvoir immunogenique a été montré non seulement in vitro sur des cellules dendritiques humaines mais aussi évalué dans le modèle de la souris transgénique HLA-A2.1 selon différents modes d'administration. D'une façon remarquable, des réponses CTL spécifiques ont été obtenues pour les dix épitopes CTL contenus dans le poly-épitope de mélanome. Les pourcentages de lyse contre les antigènes du mélanome sont également plus élevés que ceux obtenus dans les même souris HHD avec d'autres stratégies vaccinales comme les lipopeptides, vaccin recombinant ou la vaccination ADN avec les pseudo-particules HBV. En conséquence, les stratégies vaccinales, basées sur les vecteurs lentiviraux à triplex sont applicables à diverses pathologies variées tumorales ou infectieuses.

### 4- BIBLIOGRAPHIE

Blomer U, Naldini L, Kafri T, Trono D, Verma IM, Gage FH. Highly efficient and sustained gene transfer in adult neurons with a lentivirus vector. J Virol. 1997 Sep; 71(9): 6641-6649.
Chameau P, F. Clavel. 1991. A single-stranded gap in human immunodeficiency virus unintegrated linear DNA defined by a central copy of the polypurine tract. J. Virol., vol 65, N° 5, 2415-2421.
Chameau P., Alizon M. and Clavel F. (1992). A second origin of plus strand synthesis is required for optimal HIV replication. J. Virol. 66: 2814-2820.
Chameau P, G. Mirambeau, P. Roux, S. Paulous, H.Buc, F.Clavel. 1994. HIV-1 reverse transcription : a termination step at the center of the genome. J.Mol.Biol., vol.241, 651-662.
Goldman MJ, Lee PS, Yang JS, Wilson JM. Lentiviral vectors for gene therapy of cystic fibrosis. Hum Gene Ther. 1997 Dec 10; 8(18): 2261-2268.
Heyman T., Agoutin B., Friant S., Wilhelm F.X., Wilhelm M.L., 1995, Plus-Strand DNA Synthesis of the Yeast Retrotransposon Ty1 is Initiated at Two Sites, PPT1 Next to the 3' LTR and PPT2 Within the pol Gene. PPT1 is sufficient for Ty1 Transposition. J. Mol. Biol., vol. 253, 291-303.
Kafri T, Blomer U, Peterson DA, Gage FH, Verma IM. Sustained expression of genes delivered directly into liver and muscle by lentiviral vectors. Nat Genet. 1997 Nov; 17(3): 314-317.
Naldini L, Blomer U, Gage FH, Trono D, Verma IM. Efficient transfer, integration, and sustained long-term expression of the transgene in adult rat brains injected with a lentiviral vector. Proc Natl Acad Sci U S A. 1996 Oct 15; 93(21): 11382-11388.
Naldini L, Blomer U, Gallay P, Ory D, Mulligan R, Gage FH, Verma IM, Trono D. In vivo gene delivery and stable transduction of nondividing cells by a lentiviral vector. Science. 1996 Apr 12; 272(5259): 263-267.
Miller AD, Rosman GJ. Improved retroviral vectors for gene transfert and expression. Biotechniques (1989), Vol 7, p 980-990.
Pascolo S., N. Bervas, J.M. Ure, A.G. Smith, F.A. Lemonnier and B. Pérarnau. 1997. HLA-A2.1-restricted education and cytolytic activity of CD8+ T lymphocytes from ß2 microglobulin (ß2m) HLA-A2.1 monochain transgenic, H-2Db, ß2m double knockout mice. J. Exp. Med. 185, 2043-2051.
Poeschla E.M., Wong Staal F., Looney D.J. Efficient transduction of nondividing human cells by feline immunodeficiency virus lentiviral vector - Nature Medicine, vol. 4, n° 3: 354-357.
Zufferey R, Nagy D, Mandel RJ, Naldini L, Trono D. Multiply attenuated lentiviral vector achieves efficient gene delivery in vivo.Nat Biotechnol. 1997 Sep; 15(9): 871-875.

## Revendications

1. Utilisation d'un polynucléotide pour la fabrication d'un plasmide vecteur recombinant destiné à la production, par cotransfection de plasmides de transcomplémentation, de particules lentivirales recombinantes dépourvues de gènes lentiviraux et destinées à la transduction dans des cellules cibles eucaryotes d'une séquence de nucléotides d'intérêt, particules dans lesquelles ledit polynucléotide est le déterminant d'import nucléaire, ledit polynucléotide étant dérivé d'un lentivirus et constitué d'une région cis-active d'initiation centrale (cPPT) contenant au moins 10 nucléotides, d'une région cis-active de terminaison (CTS) contenant au moins 10 nucléotides, et d'un enchaînement interne de nucléotides encadré par ces deux régions, ladite région CTS et ledit enchaînement interne de nucléotides constituant une séquence ayant une taille de 80 à 120 nucléotides, ledit polynucléotide étant capable de former une structure d'ADN triplex lors de la rétrotranscription du génome desdites particules dans la cellule cible eucaryote, ledit plasmide étant dépourvu de gènes lentiviraux et comprenant une séquence de nucléotides recombinant comprenant :
- ladite séquence de nucléotides d'intérêt placée sous le contrôle de signaux régulateurs de transcription et d'expression,
- des signaux régulateurs de rétrotranscription, d'expression et d'encapsidation dérivés d'un lentivirus et
- ledit polynucléotide, les susdites régions cis-actives étant insérées dans une orientation fonctionnelle avec les susdits signaux régulateurs dérivés d'un lentivirus.

2. Utilisation selon la revendication 1, **caractérisée en ce que** le plasmide vecteur recombinant est destiné à être transcomplémenté par l'apport de séquences codant pour les polypeptides GAG, POL et ENV, ou pour une partie de ces polypeptides suffisante pour permettre la formation de particules rétrovirales.

3. Utilisation selon l'une quelconque des revendications 1 ou 2, **caractérisée en ce que** le polynucléotide est dérivé du virus HIV, CAEV, EIAV, VISNA, SIV ou FIV.

4. Utilisation selon l'une quelconque des revendications 1 à 3, **caractérisée en ce que** le polynucléotide est dérivé du génome d'un lentivirus HIV-1 ou HIV-2.

5. Utilisation selon la revendication 4, dans laquelle le polynucléotide est un fragment issu du génome de HIV-1.

6. Utilisation selon l'une quelconque des revendications 1 à 5, **caractérisée en ce que** la séquence de nucléotides d'intérêt est un gène, une partie d'un gène, une séquence dérivée d'un gène ou une séquence antisens.

## Patentansprüche

1. Verwendung eines Polynukleotids zur Herstellung eines rekombinanten Plasmidvektors, der zur Produktion durch Kotransfektion zur Transkomplementationsplamiden von rekombinanten Lentiviruspartikeln bestimmt ist, die keine lentiviralen Gene aufweisen und zur Transduktion in eukaryontischen Zielzellen von einer Nukleotidsequenz von Interesse bestimmt sind, wobei in den Partikeln das Polynukleotid die Determinante des Kernimports ist, wobei das Polynukleotid von einem Lentivirus abgeleitet ist und aus einer cis-agierenden zentralen Initiationsregion (cPPT), die mindestens 10 Nukleotide enthält, aus einer cis-agierenden Terminationsregion (CTS), die mindestens 10 Nukleotide enthält, und aus einer inneren Verkettung von Nukleotiden, die von diesen zwei Regionen flankiert ist, gebildet ist, wobei die CTS-Region und die innere Verkettung von Nukleotiden eine Sequenz bildet, die eine Größe von 80 bis 120 Nukleotiden aufweist, wobei das Polynukleotid eine Triplex-DNA-Struktur während der Retrotranskription des Genoms der Partikel in der eukaryontische Zielzelle bilden kann, wobei das Plasmid keine lentiviralen Gene aufweist und eine rekombinante Nukleotidsequenz aufweist, umfassend:
- die Nukleotidsequenz von Interesse, die unter die Kontrolle von regulatorischen Signalen von Transkription und Expression gestellt ist,
- regulatorische Signale von Retrotranskription, Expression und Encapsidation, die von einem Lentivirus abgeleitet sind, und
- das Polynukleotid, wobei die oben genannten cis-agierenden Regionen in einer funktionellen Orientierung mit den oben genannten regulatorischen Signalen, die von einem Lentivirus abgeleitet sind, eingesetzt sind.

2. Verwendung nach Anspruch 1, **dadurch gekennzeichnet, dass** der rekombinante Plasmidvektor dazu bestimmt ist, durch die Einbringung von codierenden Sequenzen für die GAG-, POL- und ENV-Polypeptide oder für einen Teil dieser Polypeptide, der ausreichend ist, um die Bildung von retroviralen Partikeln zu ermöglichen, transkomplementiert zu werden.

3. Verwendung nach irgendeinem der Ansprüche 1 oder 2, **dadurch gekennzeichnet, dass** das Polynukleotid von dem HIV-, CAEV-, EIAV-, VISNA-, SIV- oder FIV-Virus abgeleitet ist.

4. Verwendung nach irgendeinem der Ansprüche 1 bis 3, **dadurch gekennzeichnet, dass** das Polynukleotid von dem Genom eines Lentivirus HIV-1 oder HIV-2 abgeleitet ist.

5. Verwendung nach Anspruch 4, wobei das Polynukleotid ein Fragment ist, das aus dem HIV1-Genom stammt ist.

6. Verwendung nach irgendeinem der Ansprüche 1 bis 5, **dadurch gekennzeichnet, dass** die Nukleotidsequenz von Interesse ein Gen, ein Teil eines Gens, eine Sequenz, die von einem Gen abgeleitet ist, oder eine Antisense-Sequenz ist.

## Claims

1. Use of a polynucleotide for the manufacturing of a recombinant vector plasmid intended for the production, by cotransfection of transcomplementation plasmids, of recombinant lentiviral particles devoid of lentiviral genes and intended for the transduction in eukaryote target cells of a nucleotide sequence of interest, said polynucleotide being the nuclear import determinant in said particles, said polynucleotide being derived from a lentivirus and made up of a cis-active central initiation region (cPPT) containing at least 10 nucleotides, a cis-active termination region (CTS) containing at least 10 nucleotides, and an internal chain of nucleotides framed by these two regions, said CTS region and said internal chain of nucleotides making up a sequence having a size of 80 to 120 nucleotides, said polynucleotide being able to form a triplex DNA structure upon retrotranscription of the genome of said particles in the eukaryote target cell, said plasmid being devoid of lentiviral genes and comprising a recombinant nucleotide sequence comprising:
- said nucleotide sequence of interest placed under the control of transcription and expression regulation signals;
- retrotranscription, expression and encapsidation regulation signals derived from a lentivirus and
- said polynucleotide, said cis-active regions being inserted in a functional orientation with said regulation signals derived from a lentivirus.

2. Use according to claim 1, **characterized in that** the recombinant vector plasmid is intended to be transcomplemented by the provision of sequences coding for the GAG, POL and ENV polypeptides, or for a fraction of these polypeptides sufficient to allow the formation of retroviral particles.

3. Use according to any one of claims 1 or 2, **characterized in that** the polynucleotide is derived from the HIV, CAEV, EIAV, VISNA, SIV or FIV virus.

4. Use according to any one of claims 1 to 3, **characterized in that** the polynucleotide is derived from the genome of an HIV-1 or HIV-2 lentivirus.

5. Use according to claim 4, wherein the polynucleotide is a fragment stemming from the genome of HIV-1.

6. Use according to any one of claims 1 to 5, **characterized in that** the nucleotide sequence of interest is a gene, a fraction of a gene, or a sequence derived from a gene or an antisense sequence.
